(19) Europäisches Patentamt
European Patent Office
Office européen des brevets

(11) EP 1 165 528 B1

(12) **FASCICULE DE BREVET EUROPEEN**

(45) Date de publication et mention de la délivrance du brevet:
**01.09.2004 Bulletin 2004/36**

(21) Numéro de dépôt: **00911009.9**

(22) Date de dépôt: **21.03.2000**

(51) Int Cl.[7]: **C07D 265/30**, C07D 413/06, A61K 31/5375, A61K 31/5377, A61P 11/00, A61P 25/00

(86) Numéro de dépôt international:
**PCT/FR2000/000695**

(87) Numéro de publication internationale:
**WO 2000/058292 (05.10.2000 Gazette 2000/40)**

(54) **NOUVEAUX DERIVES DE MORPHOLINE, PROCEDE POUR LEUR PREPARATION ET COMPOSITIONS PHARMACEUTIQUES LES CONTENANT**

MORPHOLINDERIVATE, VERFAHREN ZU IHRER HERSTELLUNG UND SIE ENTHALTENDE PHARMAZEUTISCHE ZUSAMMENSETZUNGEN

NOVEL MORPHOLINE DERIVATIVES, METHOD FOR THE PRODUCTION THEREOF AND PHARMACEUTICAL PREPARATIONS CONTAINING SAID DERIVATIVES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **25.03.1999 FR 9903854**

(43) Date de publication de la demande:
**02.01.2002 Bulletin 2002/01**

(73) Titulaire: **SANOFI-SYNTHELABO**
**75013 Paris (FR)**

(72) Inventeurs:
- **DUCOUX, Jean, Philippe**
  **F-34090 Montpellier (FR)**
- **EMONDS-ALT, Xavier**
  **F-34980 Combaillaux (FR)**
- **GUEULE, Patrick**
  **F-34820 Teyran (FR)**
- **PROIETTO, Vincenzo**
  **F-34680 Saint Georges d'Orques (FR)**

(74) Mandataire: **Thouret-Lemaitre, Elisabeth**
**Sanofi-Synthélabo**
**Service Brevets**
**174, avenue de France**
**75013 Paris (FR)**

(56) Documents cités:
**EP-A- 0 776 893**
**WO-A-96/23787**
**WO-A-99/28307**

- **CHEMICAL ABSTRACTS, vol. 130, no. 17, 26 avril 1999 (1999-04-26) Columbus, Ohio, US; abstract no. 218281, NISHI T. ET AL: "Saturated heterocyclic compounds as substance P and neurokinin A receptor antagonists for treatment of tachykinin-related diseases" XP002140470 & JP 11 043435 A (SANKYO CO., LTD.) 16 février 1999 (1999-02-16)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet de nouveaux dérivés de morpholine, un procédé pour leur préparation et les compositions pharmaceutiques en contenant en tant que principe actif.
Plus particulièrement, la présente invention concerne de nouveaux dérivés de morpholine à usage thérapeutique, dans les phénomènes pathologiques qui impliquent le système des tachykinines comme par exemple de manière non limitative : la douleur (L. Urban et al., TINS, 1994, 17, 432-438 ; L. Seguin et al., Pain, 1995, 61, 325-343 ; S. H. Buck, 1994, The Tachykinin Receptors, Humana Press, Totowa, New-Jersey), l'allergie et l'inflammation (S. H. Buck, 1994, The Tachykinin Receptors, Humana Press, Totowa, New-Jersey), les troubles gastro-intestinaux (P. Holzer and U. Holzer-Petsche, Phamacol. Ther., 1997, 73, 173-217 et 219-263), les troubles respiratoires (J. Mizrahi et al., Pharmacology, 1982, 25, 39-50 ; C. Advenier et al., Eur. Respir. J., 1997, 10, 1892-1906 ; C. Advenier and X. Emonds-Alt, Pulmonary Pharmacol., 1996, 9, 329-333), les troubles urinaires (S. H. Buck, 1994, The Tachykinin Receptors, Humana Press, Totowa, New-Jersey ; C. A. Maggi, Progress in Neurobiology, 1995, 45, 1-98), les troubles neurologiques, les troubles neuropsychiatriques (C. A. Maggi et al., J. Autonomic Pharmacol., 1993, 13, 23-93 ; M. Otsuka and K. Yoshioka, Physiol. Rev. 1993, 73, 229-308).
Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.
Les récepteurs $NK_1$, $NK_2$, $NK_3$ ont été mis en évidence chez différentes espèces.
Une revue de C. A. Maggi et al. (J. Autonomic Pharmacol., 1993, 13, 23-93) et une revue de D. Regoli et al. (Pharmacol. Rev., 1994, 46, 551-599) font le point sur les récepteurs aux tachykinines et leurs antagonistes et exposent les études pharmacologiques et les applications en thérapeutique humaine.
De nombreux brevets ou demandes de brevet décrivent des composés actifs sur les récepteurs des tachykinines. Ainsi la demande internationale WO 96/23787 concerne les composés de formule :

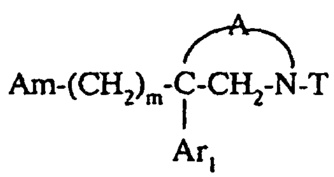

(A)

dans laquelle notamment :

- A peut représenter le radical bivalent $-O-CH_2-CH_2-$ ;
- Am, m, $Ar_1$ et T ont différentes valeurs.

La demande de brevet EP-A-0 776 893 concerne les composés de formule :

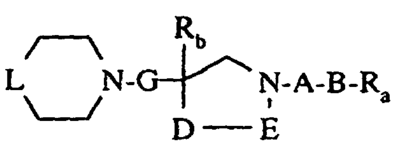

(B)

dans laquelle notamment :

- D-E peut représenter un radical bivalent $-O-CH_2-CH_2-$ ;
- L, G, E, A, B, $R_a$ et $R_b$ ont différentes valeurs.

La demande JP 11 043435A concerne des composés hétérocycliques saturés qui sont des antagonistes à la fois des récepteurs de la substance P et des récepteurs de la neurokinine A.
On a maintenant trouvé de nouveaux composés qui présentent une très forte affinité et une grande sélectivité pour les récepteurs $NK_1$ humains de la substance P et qui sont des antagonistes desdits récepteurs.

De plus, les composés selon la présente invention présentent une bonne biodisponibilité lorsqu'ils sont administrés par la voie orale.

Ces composés peuvent être utilisés pour la préparation de médicaments utiles dans le traitement de toute pathologie où la substance P et/ou les récepteurs $NK_1$ sont impliqués, notamment dans le traitement des pathologies des systèmes respiratoire, gastro-intestinal, urinaire, immunitaire, cardiovasculaire, nerveux central ainsi que dans le traitement de la douleur, de la migraine, des inflammations, des nausées et des vomissements, des maladies de la peau.

Ainsi, selon un de ses aspects, la présente invention a pour objet des composés de formule :

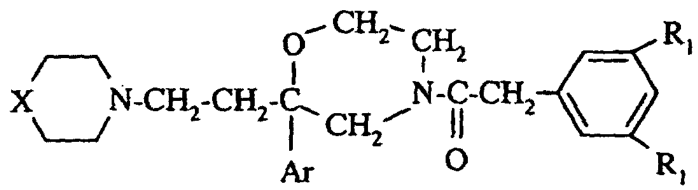

(I)

dans laquelle :

- Ar représente un phényle monosubstitué ou disubstitué par un atome d'halogène ; un $(C_1-C_3)$alkyle ;
- X représente un groupe

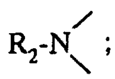

un groupe

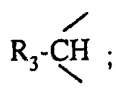

- $R_1$ représente un atome de chlore, un atome de brome, un $(C_1-C_3)$alkyle ou un trifluorométhyle ;
- $R_2$ représente un $(C_1-C_6)$alkyle ; un $(C_3-C_6)$cycloalkyle ; un groupe $-CR_4R_5CONR_6R_7$ ;
- $R_3$ représente un groupe $-CR_4R_5CONR_6R_7$ ;
- $R_4$ et $R_5$ représentent le même radical choisi parmi un méthyle, un éthyle, un n-propyle ou un n-butyle ;
- ou bien $R_4$ et $R_5$ ensemble avec l'atome de carbone auquel ils sont liés constituent un $(C_3-C_6)$cycloalkyle ;
- $R_6$ et $R_7$ représentent chacun indépendamment un hydrogène ; un $(C_1-C_3)$alkyle ;
- ou bien $R_6$ et $R_7$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

ainsi que leurs sels éventuels avec des acides minéraux ou organiques, leurs solvats et/ou leurs hydrates.

Les composés de formule (I) selon l'invention comprennent aussi bien les isomères optiquement purs que leurs mélanges en proportions quelconques.

On peut former des sels des composés de formule (I). Ces sels comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables, tels que le chlorhydrate, le bromhydrate, le sulfate, l'hydrogénosulfate, le dihydrogénophosphate, le méthanesulfonate, le méthylsulfate, l'oxalate, le maléate, le fumarate, le succinate, le naphtalène-2 sulfonate, le gluconate, le citrate, l'iséthionate, le benzènesulfonate, le paratoluènesulfonate.

Par atome d'halogène, on entend un atome de chlore, de brome, de fluor ou d'iode.

Dans la présente description les groupes alkyles sont droits ou ramifiés.

Selon la présente invention on préfère les composés de formule (I) dans laquelle Ar représente un 3,4-dichlorophényle.

Selon la présente invention, on préfère les composés de formule (I) dans laquelle les substituants $R_1$ repré-

sentent un atome de chlore, un méthyle, un éthyle, un isopropyle ou un trifluorométhyle.

Selon la présente invention, on préfère les composés de formule (I) dans laquelle X représente un groupe

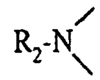

dans lequel $R_2$ représente un $(C_1-C_6)$alkyle ou un $(C_3-C_6)$cycloalkyle. Particulièrement, on préfère les composés dans lesquels $R_2$ représente un cyclopentyle ou un cyclohexyle.

Selon la présente invention, on préfère les composés de formule (I) dans laquelle X représente un groupe

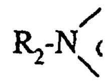

dans lequel $R_2$ représente un groupe $-CR_4R_5CONR_6R_7$.

Dans ce cas, on préfère les composés dans lesquels $R_4$ et $R_5$ représentent chacun un méthyle ou bien ensemble avec l'atome de carbone auquel ils sont liés, constituent un cyclohexyle. Particulièrement, on préfère également les composés dans lesquels $R_6$ et $R_7$ sont semblables et représentent l'hydrogène ou un méthyle.

Selon la présente invention, on préfère les composés de formule (I) dans laquelle X représente un groupe

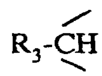

dans lequel $R_3$ représente un groupe $-CR_4R_5CONR_6R_7$.

Dans ce cas, on préfère les composés dans lesquels $R_4$ et $R_5$ représentent chacun un méthyle ou bien, ensemble avec l'atome de carbone auquel ils sont liés, constituent un cyclopropyle ou un cyclohexyle. Particulièrement, on préfère également les composés dans lesquels $R_6$ et $R_7$ sont semblables et représentent l'hydrogène ou un méthyle.

Selon la présente invention, on préfère les composés de formule:

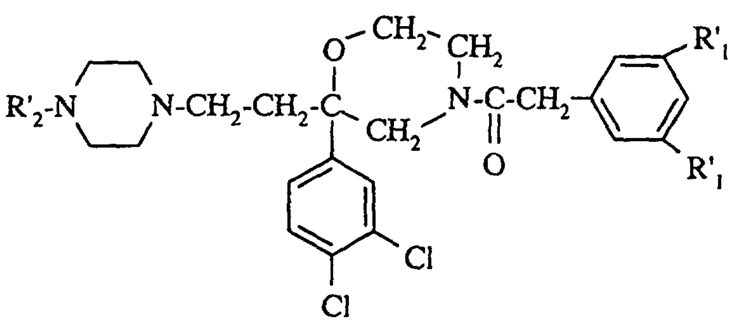

(I')

dans laquelle :

- $R'_1$ représente un atome de chlore, un méthyle, un éthyle, un isopropyle ou un trifluorométhyle ;
- $R'_2$ représente un cyclopentyle ou un cyclohexyle ;

ainsi que leurs sels avec des acides minéraux ou organiques, leurs solvats et/ou leurs hydrates.

Selon la présente invention, on préfère les composés de formule :

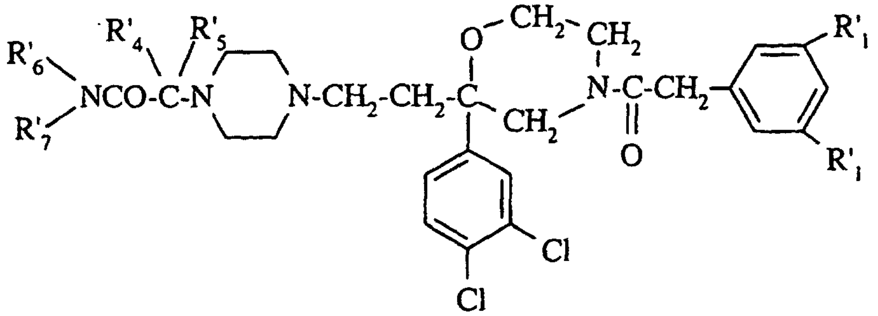

(I")

dans laquelle :

- R'$_1$ représente un atome de chlore, un méthyle, un éthyle, un isopropyle ou un trifluorométhyle ;
- R'$_4$ et R'$_5$ représentent chacun un méthyle ou bien, ensemble avec l'atome de carbone auquel ils sont liés constituent un cyclohexyle ;
- R'$_6$ et R'$_7$ sont semblables et représentent l'hydrogène ou un méthyle ;

ainsi que leurs sels avec des acides minéraux ou organiques, leurs solvats et/ou leurs hydrates.

Selon la présente invention, on préfère les composés de formule :

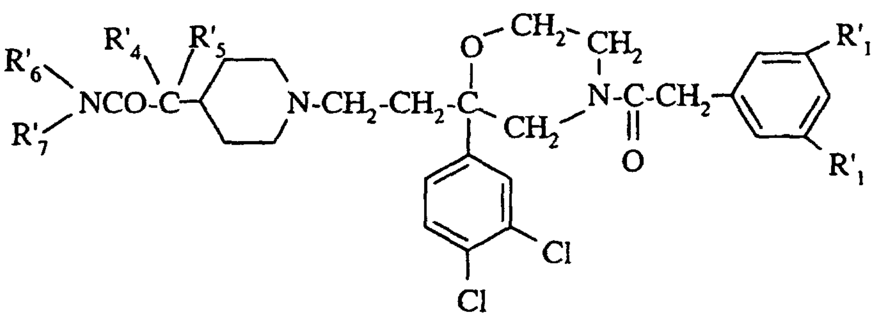

(I"')

dans laquelle :

- R'$_1$ représente un atome de chlore, un méthyle, un éthyle, un isopropyle ou un trifluorométhyle ;
- R'$_4$ et R'$_5$ représentent chacun un méthyle ou bien, ensemble avec l'atome de carbone auquel ils sont liés, constituent un cyclopropyle ou un cyclohexyle ;
- R'$_6$ et R'$_7$ sont semblables et représentent l'hydrogène ou un méthyle ;

ainsi que leurs sels avec des acides minéraux ou organiques, leurs solvats et/ou leurs hydrates.

Selon la présente invention, on préfère les composés de formule (I), (I'), (I") ou (I"') sous forme optiquement pure.

Les composés suivants :

- la 2-[2-(4-cyclohexylpipérazin-1-yl)éthyl]-2-(3,4-dichlorophényl)-4-[2-(3,5-diméthylphényl)acétyl]morpholine, isomère (-) ;
- la 2-[2-(4-cyclohexylpipérazin-1-yl)éthyl]-2-(3,4-dichlorophényl)-4-[2-(3,5-dichlorophényl)acétyl]morpholine, isomère (+) ;
- la 2-[2-(4-cyclohexylpipérazin-1-yl)éthyl]-2-(3,4-dichlorophényl)-4-[2-(3,5-bis(trifluorométhyl)phényl]acétyl]morpholine, isomère (+) ;
- la 2-[2-[4-(1-carbamoyl-1-méthyléthyl)pipéridin-1-yl]éthyl]-2-(3,4-dichlorophényl)-4-[2-(3,5-diméthylphényl)acétyl] morpholine, isomère (-) ;
- la 2-[2-[4-(1-carbamoyl-1-méthyléthyl)pipéridin-1-yl]éthyl]-2-(3,4-dichlorophényl)-4-[2-(3,5-diéthylphényl)acétyl] morpholine, isomère (-) ;
- la 2-[2-[4-(1-carbamoylcyclohexyl)pipéridin-1-yl]éthyl]-2-(3,4-dichlorophényl)-4-[2-[3,5-bis(trifluorométhyl)phényl] acétyl]morpholine, isomère (+) ;

- la 2-[2-[4-(1-carbamoyl-1-méthyléthyl)pipérazin-1-yl]éthyl]-2-(3,4-dichloro phényl)-4-[2-(3,5-diméthylphényl)acétyl]morpholine, isomère (-) ;
- la 2-[2-[4-(1-carbamoylcyclohexyl)pipérazin-1-yl]éthyl]-2-(3,4-dichlorophényl)-4-[2-(3,5-diméthylphényl)acétyl] morpholine, isomère (-) ;
- la 2-[2-[4-(1-carbamoyl-1-méthyléthyl)pipérazin-1-yl]éthyl]-2-(3,4-dichloro phényl)-4-[2-(3,5-diéthylphényl)acétyl] morpholine, isomère (-) ;
- la 2-[2-[4-(1-N,N-diméthylcarbamoyl-1-méthyléthyl)pipérazin-1-yl]éthyl]-2-(3,4-dichlorophényl)-4-[2-(3,5-diéthylphényl)acétyl]morpholine, isomère (-) ;
- la 2-[2-(4-cyclohexylpipérazin-1-yl)éthyl]-2-(3,4-dichlorophényl)-4-[2-(3,5-diéthylphényl)acétyl]morpholine, isomère (-) ;
- la 2-[2-[4-(1-carbamoylcyclopropyl)pipéridin-1-yl]éthyl]-2-(3,4-dichlorophényl)-4-[2-(3,5-diméthylphényl)acétyl] morpholine, isomère (-) ;
- la 2-[2-[4-(1-carbamoyl-1-méthyléthyl)pipérazin-1-yl]éthyl]-2-(3,4-dichlorophényl)-4-[2-(3,5-bis(trifluorométhyl) phényl]acétyl]morpholine, isomère (+) ;
- la 2-[2-[4-(1-carbamoyl-1-méthyléthyl)pipéridin-1-yl]éthyl]-2-(3,4-dichlorophényl)-4-[2-(3,5-dichlorophényl)acétyl] morpholine, isomère (+) ;
- la 2-[2-[4-(1-carbamoyl-1-méthyléthyl)pipéridin-1-yl]éthyl]-2-(3,4-dichlorophényl)-4-[2-(3,5-bis(trifluorométhyl) phényl]acétyl]morpholine, isomère (+) ;
- la 2-[2-[4-(1-carbamoyl-1-méthyléthyl)pipérazin-1-yl]éthyl]-2-(3,4-dichlorophényl)-4-[2-(3,5-diisopropylphényl) acetyl]morpholine, isomère (-) ;

ainsi que leurs sels, leurs solvats et/ou leurs hydrates sont plus particulièrement préférés.

Selon un autre de ses aspects, la présente invention concerne un procédé pour la préparation des composés de formule (I), de leurs sels, leurs solvats et/ou leurs hydrates, caractérisé en ce que :

1a) on traite un composé de formule :

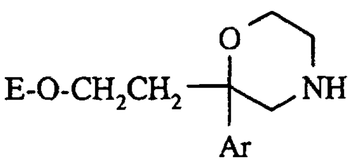

(II)

dans laquelle Ar est tel que défini pour un composé de formule (I) et E représente l'hydrogène ou un groupe O-protecteur, avec un dérivé fonctionnel d'un acide de formule :

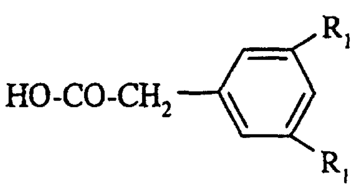

(III)

dans laquelle $R_1$ est tel que défini pour un composé de formule (I), pour obtenir un composé de formule :

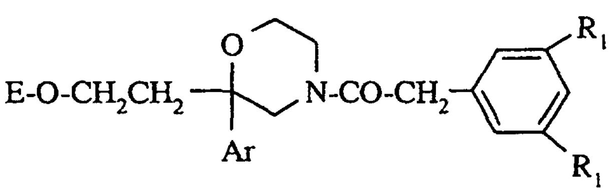

(IV)

2a) éventuellement, lorsque E représente un groupe protecteur, on l'élimine par action d'un acide ou d'une base, pour obtenir l'alcool de formule :

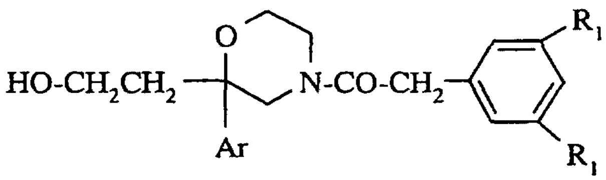

(IV, E = H)

3a) on traite l'alcool obtenu à l'étape 1a) ou à l'étape 2a) de formule (IV, E = H) avec un composé de formule :

$$Y\text{-}SO_2\text{-}Cl \qquad (V)$$

dans laquelle Y représente un groupe méthyle, phényle, tolyle, trifluorométhyle, pour obtenir un composé de formule :

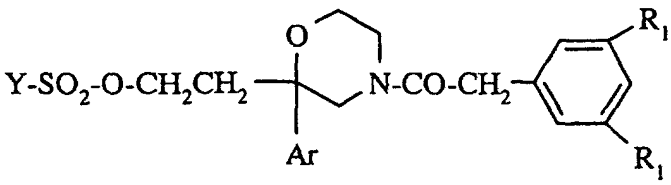

(VI)

4a) on fait réagir le composé de formule (VI) avec un composé de formule :

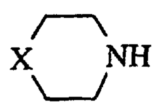

(VII)

dans laquelle X est tel que défini pour un composé de formule (I) ;

5a) et, éventuellement, on transforme le composé ainsi obtenu en l'un de ses sels avec un acide minéral ou organique.

Lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un ($C_1$-$C_4$)alkylcarbonyle.

Dans l'étape 1a), comme dérivé fonctionnel de l'acide (III), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (III) lui-même, on opère en présence d'un agent de couplage utilisé en chimie peptidique tel que le 1,3-dicyclo-hexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Le composé de formule (IV) ainsi obtenu est éventuellement déprotégé à l'étape 2a) selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe ($C_1$-$C_4$)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans

un solvant inerte tel que l'eau, le méthanol, l'éthanol, le dioxane ou un mélange de ces solvants, à une température comprise entre 0°C et la température de reflux du solvant.

A l'étape 3a) la réaction de l'alcool de formule (IV, E = H) avec un chlorure de sulfonyle de formule (V) s'effectue en présence d'une base telle que la triéthylamine, la pyridine, la N,N-diisopropyléthylamine ou la N-méthylmorpholine, dans un solvant inerte tel que le dichlorométhane, le benzène ou le toluène, à une température comprise entre -20°C et la température de reflux du solvant.

Le composé de formule (VI) ainsi obtenu est mis en réaction à l'étape 4a), avec un composé de formule (VII). La réaction s'effectue dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (VII) et en présence d'un iodure de métal alcalin tel que l'iodure de potassium ou l'iodure de sodium. La réaction s'effectue à une température comprise entre la température ambiante et 100°C.

Selon une variante du procédé :

1b) on procède comme à l'étape 1a) et éventuellement à l'étape 2a) ;
2b) on oxyde le composé de formule (IV, E = H) ainsi obtenu pour préparer un composé de formule :

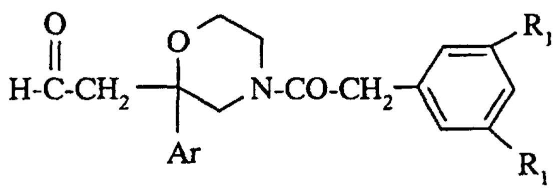

(VIII) ;

3b) on fait réagir le composé de formule (VIII) avec un composé de formule (VII) tel que défini précédemment, en présence d'un acide, puis on réduit le sel d'imminium formé intermédiairement au moyen d'un agent réducteur ;
4b) et, éventuellement, on transforme le composé ainsi obtenu en un de ses sels avec un acide minéral ou organique.

Selon la variante du procédé, à l'étape 2b) on soumet un alcool de formule (IV, E = H) à une oxydation pour obtenir un aldéhyde de formule (VIII). La réaction d'oxydation s'effectue en utilisant par exemple le chlorure d'oxalyle, le diméthylsulfoxyde et la triéthylamine dans un solvant tel que de dichlorométhane et à une température comprise entre -78°C et la température ambiante.

Puis à l'étape 3b), on fait réagir le composé de formule (VII) avec un aldéhyde de formule (VIII) en présence d'un acide tel que l'acide acétique, dans un solvant inerte tel que le méthanol ou le dichlorométhane, pour former *in-situ* une imine intermédiaire qui est réduite chimiquement en utilisant par exemple le cyanoborohydrure de sodium ou le triacétoxyborohydrure de sodium, ou catalytiquement en utilisant l'hydrogène et un catalyseur tel que le palladium sur charbon ou le nickel de Raney®.

Selon une autre variante du procédé :

1c) on protège l'atome d'azote d'un composé de formule (II) par un groupe N-protecteur pour obtenir un composé de formule :

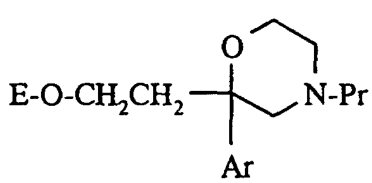

(XXXV)

dans laquelle Ar est tel que défini pour un composé de formule (I), E représente l'hydrogène ou un groupe O-protecteur et Pr représente un groupe N-protecteur ;
2c) éventuellement, lorsque E représente un groupe protecteur, on l'élimine par action d'un acide ou d'une base, pour obtenir l'alcool de formule :

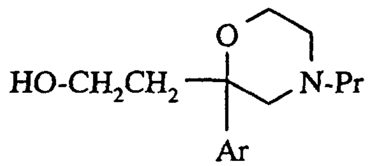

(XXXV, E = H)

3c) on traite l'alcool obtenu à l'étape 1c) ou à l'étape 2c) de formule (XXXV, E = H) avec un composé de formule :

$$Y\text{-}SO_2\text{-}Cl \quad (V)$$

dans laquelle Y représente un groupe méthyle, phényle, tolyle, trifluorométhyle, pour obtenir un composé de formule :

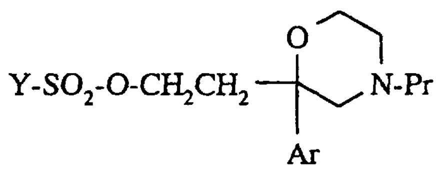

(XXXVI)

4c) on fait réagir le composé de formule (XXXVI) avec un composé de formule:

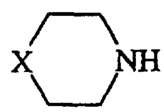

(VII)

dans laquelle X est tel que défini pour un composé de formule (I), pour obtenir un composé de formule :

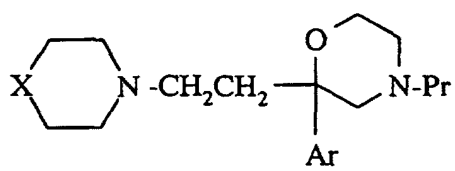

(XXXVII)

5c) on élimine le groupe N-protecteur du composé de formule (XXXVII) pour obtenir un composé de formule :

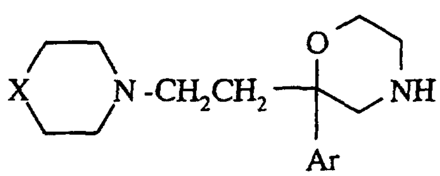

(XXXVIII)

6c) on traite le composé de formule (XXXVIII) avec un dérivé fonctionnel d'un acide de formule :

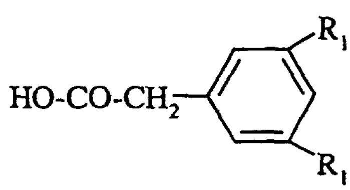

(III)

dans laquelle $R_1$ est tel que défini pour un composé de formule (I) ;
7c) et, éventuellement, on transforme le composé ainsi obtenu en l'un de ses sels avec un acide minéral ou organique.

Lorsque Pr représente un groupe N-protecteur, celui-ci est choisi parmi les groupes N-protecteurs classiques bien connus de l'homme de l'art, tels que, par exemple, le groupe *tert*-butoxycarbonyle, le benzyloxycarbonyle, le trityle ou le benzyle.

On obtient finalement les composés de formule (I) selon l'invention.

Les composés de formule (I) ainsi obtenus sont isolés sous forme de base libre ou de sel, selon les techniques classiques.

Lorsque les composés de formule (I) sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, ou dans l'acétate d'éthyle avec une solution de l'acide choisi dans un des solvants précités, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le succinate, le fumarate, le naphtalène-2-sulfonate, le benzènesulfonate, le para-toluènesulfonate, le gluconate, le citrate, l'iséthionate.

A la fin de la réaction, les composés de formule (I) peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) dans laquelle E représente l'hydrogène ou un groupe O-protecteur se préparent selon les SCHEMAS 1 et 2 ci-après dans lesquels $Pr_1$ et $Pr_2$ représentent un groupe O-protecteur tel que défini ci-dessus pour E, plus particulièrement $Pr_1$ représente un groupe O-protecteur hydrolysable en milieu acide, $Pr_2$ représente un groupe O-protecteur hydrolysable en milieu basique.

## SCHEMA 1

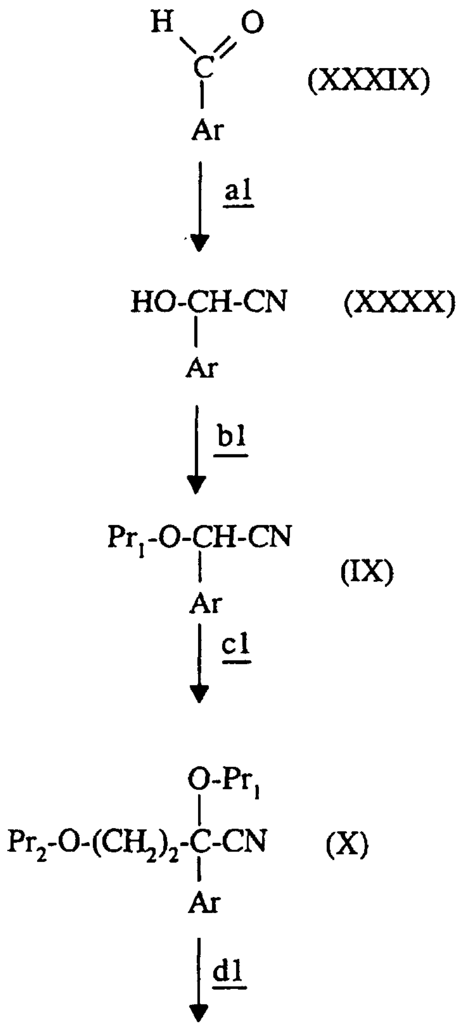


(XXXIX)

(XXXX)

(IX)

(X)

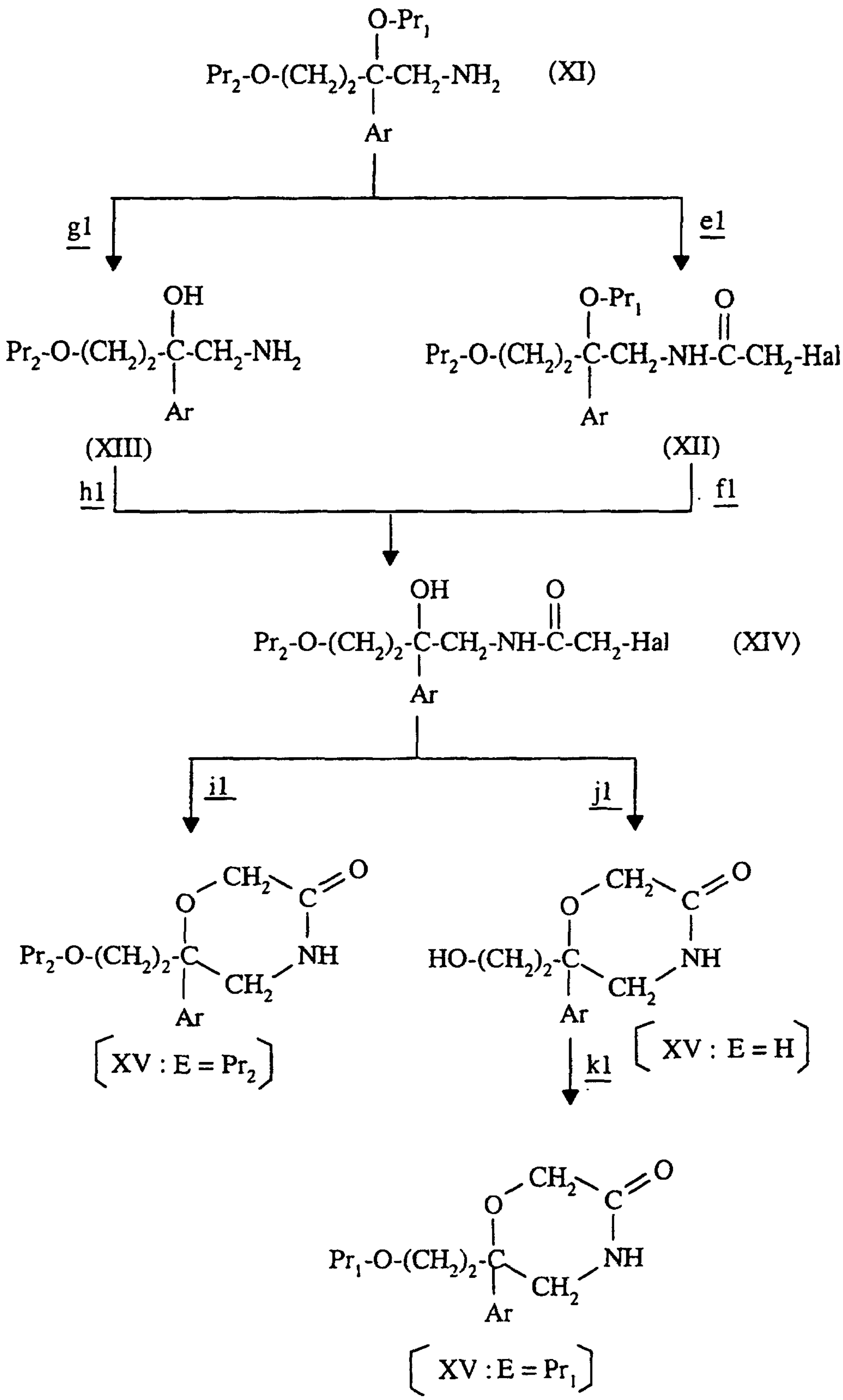


(XI)

(XIII)

(XII)

(XIV)

[XV : E = Pr2]

[XV : E = H]

[XV : E = Pr1]

SCHEMA 2

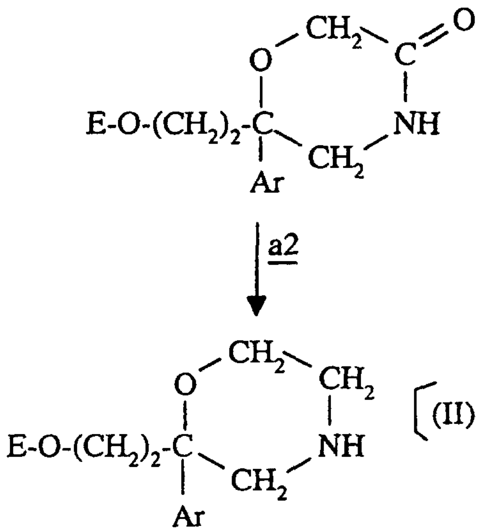


[XV : E = H, $Pr_1$ ou $Pr_2$]

[(II) : E = H, $Pr_1$ ou $Pr_2$]

A l'étape a1 du SCHEMA 1, la synthèse d'une cyanhydrine (XXXX) à partir d'un aldéhyde (XXXIX) s'effectue selon les méthodes bien connues de l'homme de l'art telle que par exemple celle décrite dans Organic Syntheses ; Wiley, New-York, 1932 ; Collect. vol. 1, p. 336, ou par adaptation de cette méthode en utilisant l'action du métabisulfite de sodium et du cyanure de potassium en solution aqueuse.

A l'étape b1, le groupe hydroxy du composé (XXXX) est protégé selon les méthodes connues de l'homme de l'art.

Le composé de formule (IX) ainsi obtenu est traité à l'étape c1 par une base forte telle que le diisopropylamidure de lithium, le *tert*-butylate de potassium ou l'hydrure de sodium pour fournir un carbanion qui est mis en réaction avec un composé de formule Hal-$(CH_2)_2$-O-$Pr_2$, dans laquelle Hal représente un halogène de préférence le brome ou le chlore, pour obtenir le composé de formule (X). La réaction s'effectue dans un solvant inerte tel qu'un éther (tétrahydrofurane, éther diéthylique, 1,2-diméthoxyéthane par exemple) ou un amide (N,N-diméthyl-formamide par exemple) ou un hydrocarbure aromatique (toluène, xylène par exemple) à une température comprise entre -70°C et +60°C.

Le dérivé nitrile de formule (X) est réduit à l'étape d1 pour obtenir l'amine primaire de formule (XI). Cette réduction peut s'effectuer au moyen d'hydrogène, en présence d'un catalyseur tel que le nickel de Raney®, dans l'éthanol en mélange avec de l'ammoniaque, ou au moyen d'un agent réducteur tel que l'hydrure d'aluminium lithium, l'hydrure de diisobutylaluminium, le borane dans le THF, dans un solvant tel que le toluène, l'hexane, l'éther de pétrole, le xylène ou le tétrahydrofurane. La réaction s'effectue à une température comprise entre 0°C et 70°C.

A l'étape e1, le composé de formule (XI) est mis en réaction, avec un composé de formule Hal-CO-$CH_2$-Hal dans laquelle Hal représente un halogène, de préférence le chlore ou le brome, en présence d'une base telle qu'une amine tertiaire (triéthylamine, N-méthylmorpholine, pyridine par exemple) pour obtenir un composé de formule (XII). La réaction s'effectue dans un solvant inerte tel qu'un solvant chloré (dichlorométhane, dichloroéthane, chloroforme par exemple), un éther (tétrahydrofurane, dioxane par exemple) ou un amide (N,N-diméthylformamide par exemple) à une température comprise entre -70°C et la température ambiante.

On élimine à l'étape f1 le groupe O-protecteur $Pr_1$ du composé de formule (XII), par hydrolyse acide selon les méthodes précédemment décrites.

Alternativement, on élimine à l'étape g1 le groupe O-protecteur $Pr_1$ du composé de formule (XI) par hydrolyse acide, puis on met en réaction, à l'étape h1, le composé (XIII) ainsi obtenu avec un composé de formule Hal-CO-$CH_2$-Hal selon les méthodes décrites ci-dessus à l'étape e1.

Le composé de formule (XIV) ainsi obtenu est cyclisé en présence d'une base pour obtenir le composé de formule (XV). Lorsqu'on veut obtenir un composé de formule (XV) dans laquelle E représente un groupe protecteur $Pr_2$, on utilise une base telle qu'un carbonate de métal alcalin (le carbonate de potassium par exemple) ou un hydrure de métal alcalin (l'hydrure de sodium par exemple) ou le *tert*-butylate de potassium, dans un solvant inerte tel qu'un hydrocarbure aromatique (xylène, toluène par exemple) ou un amide (N,N-diméthylformamide par exemple) ou un éther (tétrahydrofurane par exemple), à une température comprise entre -30°C et la température de reflux du solvant (étape i1). Lorsqu'on veut obtenir un composé de formule (XV) dans laquelle E représente l'hydrogène, on utilise une base telle qu'un hydroxyde de métal alcalin (l'hydroxyde de sodium, l'hydroxyde de potassium par exemple) en solution

aqueuse concentré dans un solvant tel qu'un alcanol (le propan-2-ol par exemple) ou un amide (le N,N-diméthylformamide par exemple) ou un mélange de ces solvants à une température comprise entre la température ambiante et la température de reflux du solvant (étape j1).

Eventuellement, on prépare à l'étape k1 un composé de formule (XV) dans laquelle E représente un groupe O-protecteur $Pr_1$ selon les méthodes connues de l'homme de l'art.

A l'étape a2 du SCHEMA 2, on réduit un composé de formule (XV) dans laquelle E représente l'hydrogène ou un groupe O-protecteur, obtenu selon le SCHEMA 1. La réduction s'effectue au moyen d'un agent réducteur tel que l'hydrure aluminium et de lithium, l'hydrure de diisobutylaluminium, le borohydrure de sodium, le borane dans le THF, dans un solvant inerte tel que le tétrahydrofurane, l'éther diéthylique, le 1,2-diméthoxyéthane ou le toluène à une température comprise entre la température ambiante et la température de reflux du solvant. On obtient ainsi le composé de formule (II) attendu. De façon particulière, lorsque dans le composé de formule (XV) E représente un groupe benzoyle, on obtient lors de la réduction, un mélange d'un composé de formule (II) dans laquelle E = H et d'un composé de formule (II) dans laquelle E = benzoyle. On sépare ces composés selon les techniques classiques, par exemple par chromatographie.

Les composés de formule (III) sont commerciaux ou préparés selon des méthodes connues.

Ainsi, par exemple, on prépare les composés de formule (III) selon le SCHEMA 3 ci-après.

SCHEMA 3

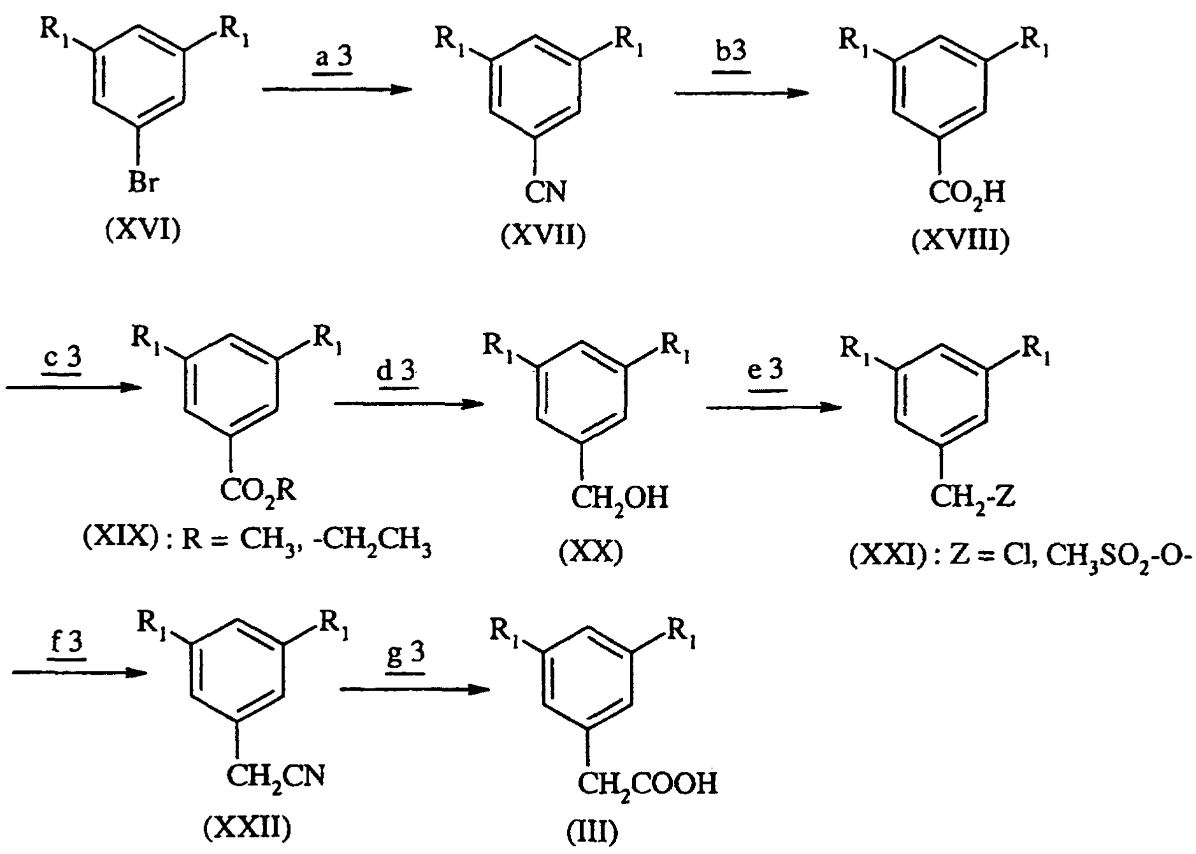


(XVI) (XVII) (XVIII)

(XIX) : R = $CH_3$, $-CH_2CH_3$ (XX) (XXI) : Z = Cl, $CH_3SO_2$-O-

(XXII) (III)

Les étapes a3 et b3 du SCHEMA 3 s'effectuent selon les méthodes décrites dans J. Am. Chem. Soc., 1941, 63, 3280-3282.

A l'étape c3 on prépare un ester de formule (XIX) à partir d'un acide de formule (XVIII) selon les méthodes connues de l'homme de l'art.

L'ester (XIX) ainsi obtenu est réduit à l'étape d3 en alcool de formule (XX) selon les méthodes connues de l'homme de l'art.

Les étapes e3 et f3 s'effectuent selon les méthodes décrites dans J. Med. Chem., 1973, 16, 684-687.

Les dérivés phénylacétonitrile de formule (XXII) ainsi obtenus sont hydrolysés en composés de formule (III) selon les méthodes décrites dans J. Org. Chem., 1968, 33, 4288 ou dans EP-A-0 714 891.

Les dérivés bromés de formule (XVI) sont connus ou préparés selon des méthodes connues telles que celles

décrites dans J. Org. Chem., 1971, 36(1), 193-196, ou dans J. Am. Chem. Soc., 1941, 63, 3280-3282.

Les composés de formule (VII) dans laquelle X représente un groupe

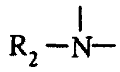

dans lequel $R_2$ représente un $(C_1\text{-}C_6)$alkyle ou un $(C_1\text{-}C_6)$cycloalkyle sont commerciaux ou préparés selon des méthodes connues telles que celles décrites dans J. Org. Chem. 1957, 22, 713 ou J. Med. Chem., 1992, 35, 2688-2696.

Les composés de formule (VII) dans laquelle X représente un groupe

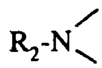

dans lequel $R_2$ représente un groupe $-CR_4R_5CONR_6R_7$ se préparent selon le SCHEMA 4 ci-après :

SCHEMA 4

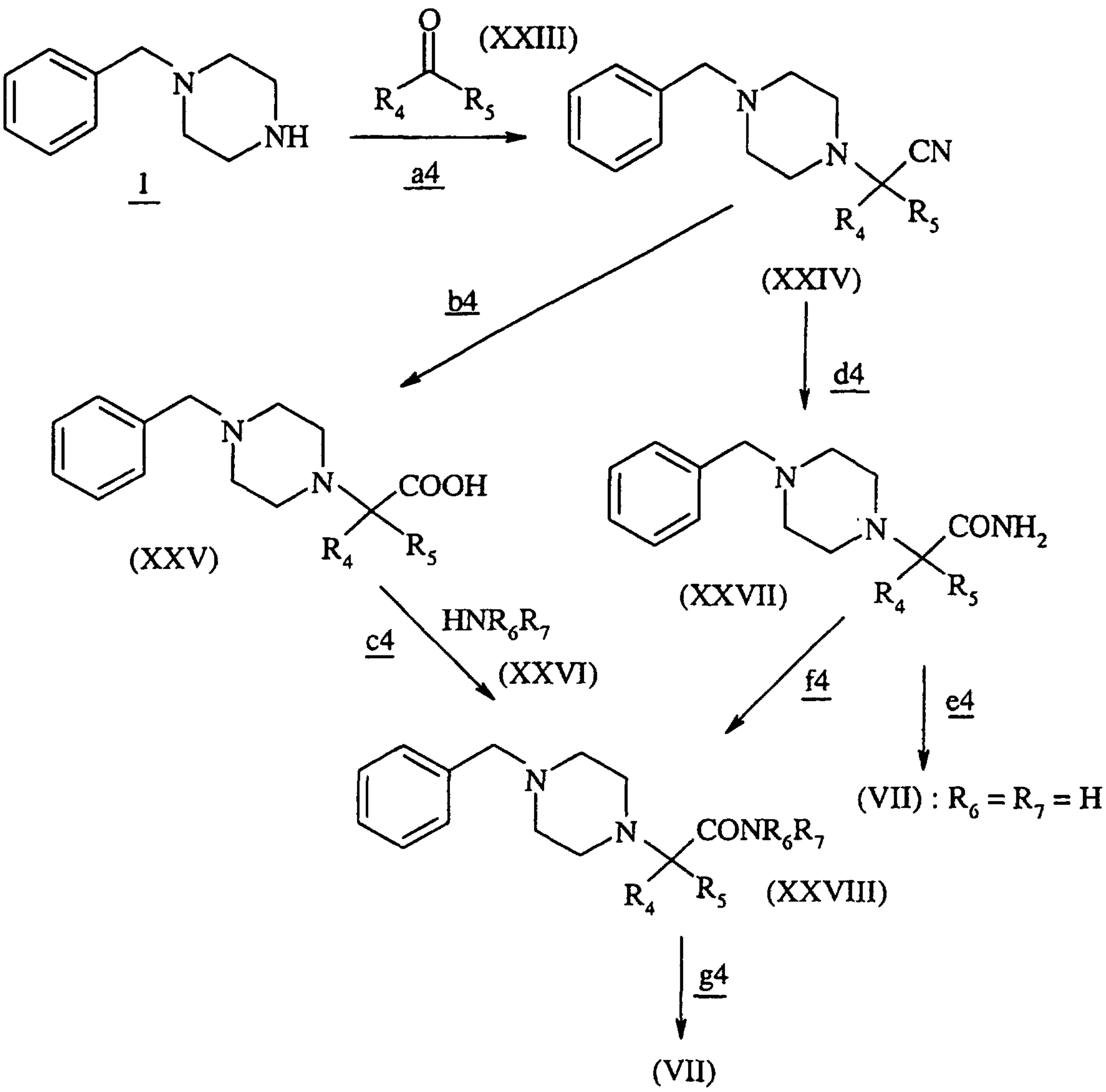


A l'étape a4 du SCHEMA 4, on fait réagir le composé 1 avec une cétone de formule (XXIII), en présence de

2-hydroxyisobutyronitrile, selon la méthode décrite dans Eur. J. Med. Chem., 1990, 25, 609-615.

Le dérivé nitrile de formule (XXIV) ainsi obtenu est hydrolysé à l'étape b4 selon les méthodes connues de l'homme de l'art pour donner un dérivé acide de formule (XXV).

L'acide (XXV) est mis en réaction à l'étape c4 avec une amine de formule (XXVI) selon les méthodes classiques du couplage peptidique pour donner le dérivé (XXVIII).

Alternativement, on hydrolyse, à l'étape d4, le dérivé nitrile de formule (XXIV) selon les méthodes connues pour obtenir le dérivé carboxamide de formule (XXVII), qui est éventuellement déprotégé à l'étape e4, selon les méthodes classiques, pour obtenir le composé (VII) dans lequel $R_6 = R_7 = H$.

A l'étape f4, par réaction du composé de formule (XXVII), en présence d'une base forte, respectivement, avec un halogènure de $(C_1\text{-}C_3)$alkyle, ou successivement avec deux halogénures de $(C_1\text{-}C_3)$alkyle, ou avec un dihalogénure de formule Hal-$R_6$-$R_7$-Hal, selon les méthodes classiques d'alkylation, on prépare un composé de formule (XXVIII) dans laquelle respectivement $R_6$ représente un $(C_1\text{-}C_3)$alkyle et $R_7$ = H, ou $R_6$ et $R_7$ représentent chacun indépendamment un $(C_1\text{-}C_3)$alkyle, ou $R_6$ et $R_7$ ensemble avec l'atome d'azote auquel ils sont liés constituent un hétérocycle.

Le composé (XXVIII) ainsi obtenu est déprotégé à l'étape g4, selon les méthodes connues, pour donner le composé (VII) attendu.

Les composés de formule (VII) dans laquelle X représente un groupe

$$-\underset{|}{C}H-CR_4R_5CONR_6R_7$$

se préparent selon le SCHEMA 5 ci-après.

SCHEMA 5

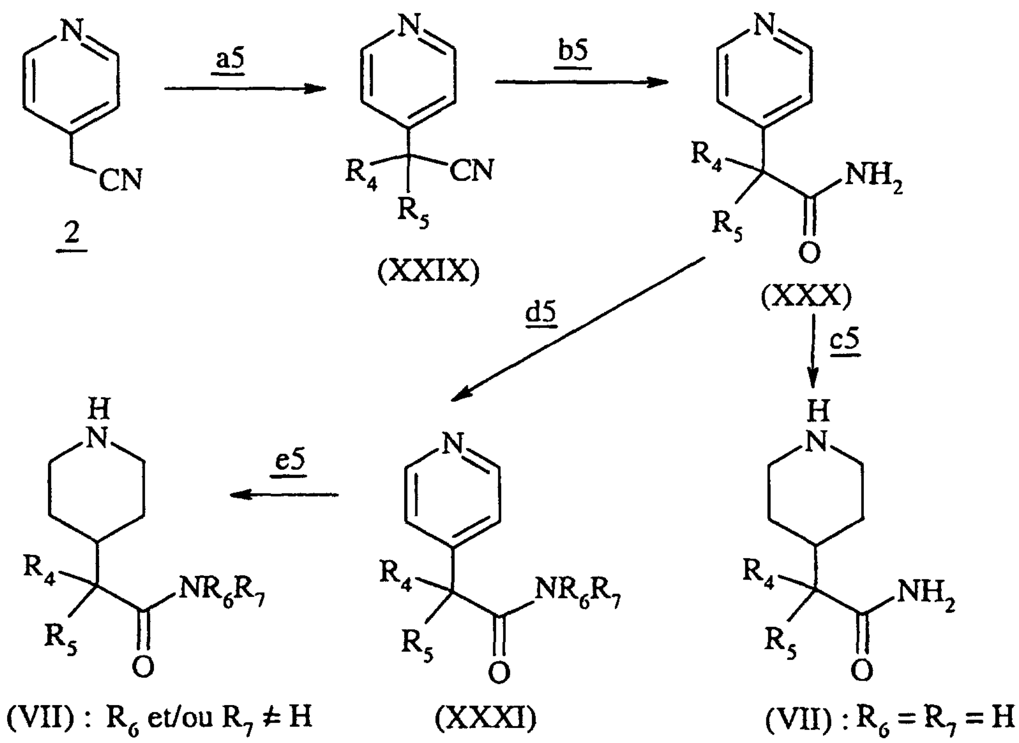


2

(XXIX)

(XXX)

(VII) : $R_6$ et/ou $R_7 \neq H$

(XXXI)

(VII) : $R_6 = R_7 = H$

A l'étape a5 du SCHEMA 5, la réaction du composé 2, en présence d'une base forte telle que l'hydrure de sodium ou l'amidure de sodium, avec respectivement un halogénure de $(C_1\text{-}C_4)$alkyle linéaire, ou avec un dihalogénure de formule Hal$(CH_2)_m$-Hal dans laquelle m = 2 à 5 et Hal représente un atome d'halogène dans un solvant inerte tel que le N,N-diméthylformamide ou le dichlorométhane et à une température comprise entre 0°C et la température ambiante, selon les méthodes classiques d'alkylation, permet d'obtenir le composé de formule (XXIX) dans laquelle, respectivement $R_4$ et $R_5$ représentent chacun un $(C_1\text{-}C_4)$alkyle linéaire ou, ensemble avec l'atome de carbone auquel ils sont liés constituent un $(C_3\text{-}C_6)$cycloalkyle.

Le dérivé nitrile (XXIX) ainsi obtenu est hydrolysé à l'étape b5 selon les méthodes connues de l'homme de l'art pour donner le dérivé carboxamide (XXX). Eventuellement, à l'étape c5, on hydrogène, en présence d'un catalyseur tel que l'oxyde de platine, le cycle pyridine selon les méthodes classiques, pour obtenir un composé de formule (VII) dans laquelle $R_6$ et $R_7$ = H.

A l'étape d5, par réaction d'alkylation, selon les méthodes classiques précédemment décrites, du composé de formule (XXX), puis réduction, par hydrogénation catalytique classique, du composé (XXXI) ainsi obtenu, on obtient un composé de formule (VII) dans laquelle $R_6$ et/ou $R_7 \neq$ H.

On peut également obtenir les composés de formule (VII) dans laquelle X représente un groupe

$$-\underset{|}{C}H-CR_4R_5CONR_6R_7$$

selon le SCHEMA 6 ci-après.

SCHEMA 6

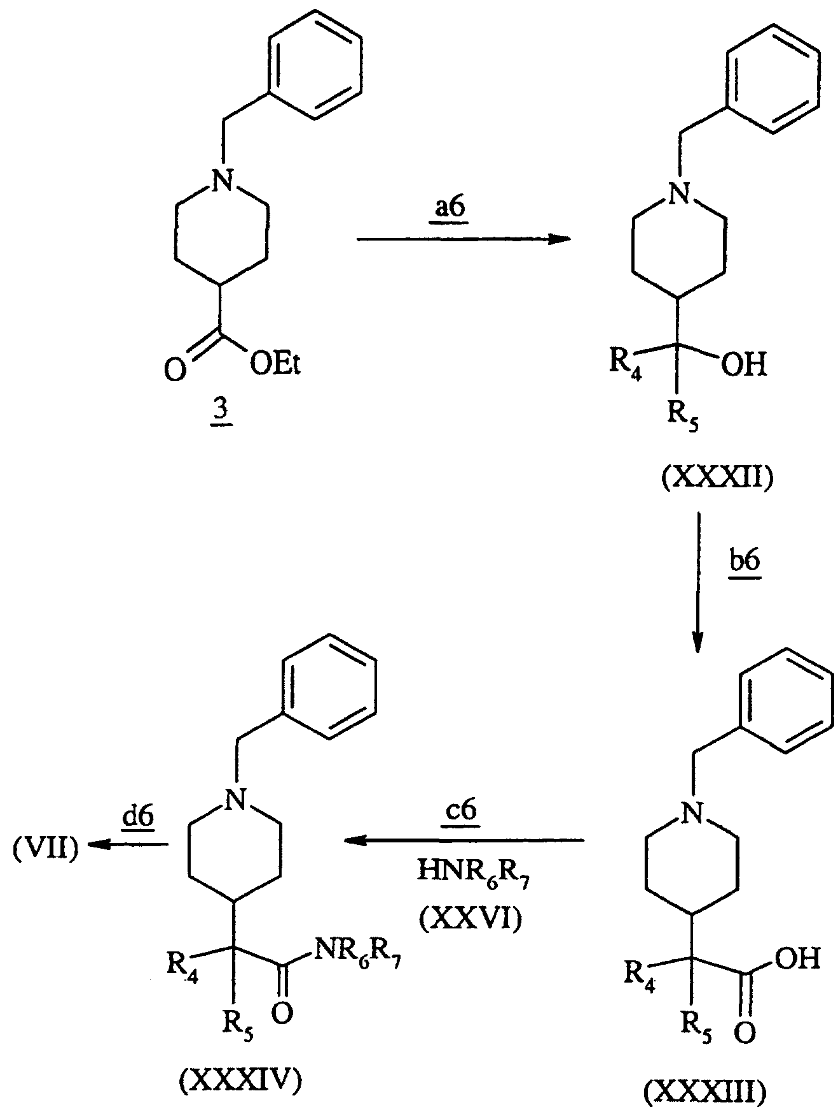

3
(XXXII)
(XXXIII)
(XXXIV)
(VII)

A l'étape a6 du SCHEMA 6, la réaction du composé 3 avec un dérivé organolithien ou organomagnésien approprié tel que, par exemple, le méthyllithium, le chlorure d'éthylmagnésium, le chlorure de propylmagnésium ou le pentane-1,5-di(magnésium chlorure), selon les méthodes décrites dans EP-A-0 625 509, permet d'obtenir l'alcool de formule (XXXII).

L'alcool (XXXII) ainsi obtenu est oxydé à l'étape b6 en acide de formule (XXXIII) selon la méthode décrite dans Helvetica Chimica Acta, 1972, 55 (7), 2439.

L'acide (XXXIII) est mis en réaction à l'étape c6 avec une amine de formule (XXVI) selon les méthodes classiques du couplage peptidique pour donner le composé (XXXIV).

Le composé (XXXIV) est déprotégé à l'étape d6, selon les méthodes connues, pour donner le composé (VII) attendu.

Le composé 3 se prépare par réaction de l'isonipécotate d'éthyle avec du bromure de benzyle, en présence d'une base, selon les méthodes classiques d'alkylation.

Au cours de l'une quelconque des étapes de préparation des composés de formule (I) ou des composés intermédiaires de formule (II), (III) ou (VII), il peut être nécessaire et/ou souhaitable de protéger les groupes fonctionnels réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utlisant les méthodes connues de l'homme de l'art et qui n'affectent pas le reste de la molécule concernée.

Les composés de formule (IV) sont préparés à l'étape 1a) du procédé selon l'invention.

Les composés de formule (VI) sont préparés à l'étape 3a) du procédé selon l'invention.

Les composés de formule (VIII) sont préparés à l'étape 2b) de la variante du procédé selon l'invention.

La résolution des mélanges racémiques des composés de formule (I) permet d'isoler les énantiomères.

Il est cependant préférable d'effectuer le dédoublement des mélanges racémiques à partir du composé de formule (II : E = H) utile pour la préparation d'un composé de formule (I) ou bien à partir d'un composé intermédiaire utile pour la préparation d'un composé de formule (II). En particulier, on effectue le dédoublement du mélange racémique du composé de formule :

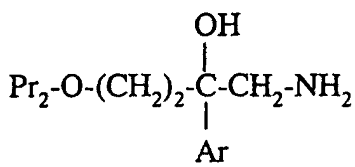

(XIII)

Lorsque le dédoublement des racémiques est effectué sur les composés intermédiaires de formule (XIII) ou (II) (E = H) celui-ci peut s'effectuer selon des méthodes connues par formation d'un sel avec des acides optiquement actifs, par exemple avec l'acide (+) ou (-) tartrique ou l'acide (+) ou (-)-10-camphorsulfonique. Les diastéréoisomères sont alors séparés par des méthodes classiques telles que la cristallisation ou la chromatographie puis, après libération de la base, on obtient les énantiomères optiquement purs.

Les composés de formule (I) ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'essais biochimiques.

L'affinité des composés pour les récepteurs aux tachykinines a été évaluée in vitro par plusieurs essais biochimiques utilisant des radioligands :

1) La liaison de [$^{125}$I] BH-SP (Substance P marquée à l'iode-125 à l'aide du réactif de Bolton-Hunter) aux récepteurs $NK_1$ des cellules lymphoblastiques humaines (D. G. Payan et al., J. Immunol., 1984, 133, 3260-3265).
2) La liaison [$^{125}$I] His-$NK_A$ aux récepteurs clonés $NK_2$ humains exprimés par des cellules CHO (Y. Takeda et al., J. Neurochem., 1992, 59, 740-745).
3) La liaison [$^{125}$I] His [$MePhe^7$] $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413 ; Life Sci., 1995, 56, PL 27-32).

Les composés selon l'invention inhibent fortement la liaison de la substance P aux récepteurs $NK_1$ des cellules lymphoblastiques humaines IM9. La constante d'inhibition Ki pour les récepteurs des cellules lymphoblastiques humaines est de l'ordre de $10^{-11}$M.

Les constantes d'inhibition Ki pour les récepteurs clonés $NK_2$ humains sont de l'ordre de $10^{-8}$M et les constantes d'inhibition Ki pour les récepteurs clonés $NK_3$ humains sont supérieures à $10^{-7}$M.

Les composés de formule (I) sont des antagonistes, puissants et sélectifs, des récepteurs $NK_1$ humains de la substance P.

Ainsi les composés de formule (I) ont également été évalué in vivo sur des modèles animaux.

Chez le cobaye, au niveau du striatum, l'application locale d'un agoniste spécifique des récepteurs $NK_1$, par exemple [$Sar^9$, $Met(O_2)^{11}$]substance P, augmente la libération d'acétylcholine. Cette libération est inhibée par l'administration orale ou intrapéritonéale des composés selon la présente invention. Ce test a été adapté de la méthode décrite par R. Steinberg et al., J. Neurochemistry, 1995, 65, 2543-2548.

Ces résultats montrent que les composés de formule (I) sont actifs par voie orale, qu'ils passent la barrière hématoméningée et qu'ils sont susceptibles de bloquer, au niveau du système nerveux central, l'action propre aux récepteurs $NK_1$.

Les composés de formule (I) ont été évalués dans le test de bronchoconstriction chez le cobaye, selon la méthode décrite par X. Emonds-Alt et al., European Journal of Pharmacology, 1993, 250, 403-413. Les composés de formule (I) administrés par voie intraveineuse antagonisent fortement la bronchoconstriction induite par administration intraveineuse de septide chez le cobaye dans ces conditions expérimentales.

L'activité pharmacologique in vivo des composés de formule (I) a également été évaluée dans le modèle d'hypotension chez le chien, selon la méthode décrite par X. Emonds-Alt et al., Eur. J. Pharmacol., 1993, 250, 403-413. Les composés de formule (I) administrés par voie intraveineuse inhibent fortement l'hypotension induite par administration intraveineuse de [$Sar^9$, $Met(O_2)^{11}$]substance P chez le chien anesthésié dans ces conditions expérimentales.

Ces résultats montrent que les composés de formule (I) bloquent au niveau du système nerveux périphérique, l'action propre aux récepteurs $NK_1$.

Les composés de la présente invention sont notamment des principes actifs de compositions pharmaceutiques, dont la toxicité est compatible avec leur utilisation en tant que médicaments.

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I) ou l'un de ses sels, solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthylcellulose, des délitants comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée

ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lesquels le principe actif peut être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, α, β, γ-cyclodextrine, 2-hydroxypropyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Selon un autre de ses aspects, la présente invention concerne l'utilisation des composés de formule (I), ou de l'un de leurs sels, solvats et/ou hydrates pharmaceutiquement acceptables pour la préparation de médicaments destinés à traiter toute pathologie où la substance P et/ou les récepteurs NK1 humains sont impliqués.

Selon un autre de ses aspects, la présente invention concerne l'utilisation des composés de formule (I) ou de l'un de leurs sels, solvats et/ou hydrates pharmaceutiquement acceptables pour la préparation de médicaments destinés à traiter les pathologies du système respiratoire, gastro-intestinal, urinaire, immunitaire, cardiovasculaire et du système nerveux central ainsi que la douleur, la migraine, les inflammations, les nausées et les vomissements, les maladies de la peau.

Par exemple et de manière non limitative, les composés de formule (I) sont utiles :

- comme analgésique, en particulier dans le traitement de douleurs traumatiques telles que les douleurs post-opératoires ; de la névralgie du plexus brachial ; de douleurs chroniques telles que les douleurs arthritiques due à l'ostéoarthrite, l'arthrite rhumatoïde ou l'arthrite psoriatique ; de douleurs neuropathiques telles que la névralgie post-herpétique, la névralgie du trijumeau, la névralgie segmentale ou intercostale, la fibromyalgie, la causalgie, la neuropathie périphérique, la neuropathie diabétique, les neuropathies induites par une chimiothérapie, les neuropathies liées au SIDA, la névralgie occipitale, la névralgie géniculée, la névralgie glossopharyngienne ; de douleurs de l'illusion des amputés ; de diverses formes de mal de tête telle que la migraine chronique ou aiguë, de

douleur temporomandibulaire, de douleur du sinus maxillaire, du névralgisme facial, d'odontalgie ; de douleur du cancéreux ; de douleur d'origine viscérale ; de douleur gastro-intestinale ; de douleur due à la compression nerveuse, de douleurs dues à la pratique intensive d'un sport ; de la dysménorrhée ; de douleur menstruelle ; de douleur due à une méningite, à une arachnoïdite ; de douleur muscosquelettique ; de douleurs du bas du dos dues à une sténose spinale, à un disque prolabé, à une sciatique ; de douleur de l'angineux ; de douleurs dues à une spondylite ankylosante ; de douleurs liées à la goutte ; de douleurs liées aux brûlures, à la cicatrisation, à une dermatose prurigineuse ; de douleur thalamique ;

- comme antiinflammatoire en particulier pour traiter les inflammations dans l'asthme, l'influenza, les bronchites chroniques (en particulier la bronchite chronique obstructive, COPD de l'anglais chronic obstructive pulmonary disease), les toux, les allergies, le bronchospasme et l'arthrite rhumatoïde ; les maladies inflammatoires du système gastro-intestinal par exemple la maladie de Crohn, les colites ulcératives, les pancréatites, les gastrites, l'inflammation des intestins, les troubles causés par les antiinflammatoires non-stéroidiens, les effets inflammatoires et secrétoires dûs aux infections bactériennes par exemple dûe au Clostridium difficile ; les maladies inflammatoires de la peau, par exemple l'herpès et l'eczéma ; les maladies inflammatoires de la vessie telles que la cystite et l'incontinence ; les inflammations ophtalmiques telles que la conjonctivite, la vitréorétinopathie ; les inflammations dentaires telles que gingivite et périodontite ;
- dans le traitement des maladies allergiques en particulier de la peau comme l'urticaire, les dermatites de contact, les dermatites atopiques et des maladies respiratoires comme les rhinites ;
- dans le traitement des maladies du système nerveux central en particulier des psychoses telles que la schizophrénie, la manie et la démence ; des désordres cognitifs telle que la maladie d'Alzheimer, l'anxiété, la démence liée au SIDA ; des neuropathies diabétiques ; de la dépression ; de la maladie de Parkinson ; de la dépendance aux drogues; des abus de substances ; des troubles de la vigilance, du sommeil, du rythme circadien, de l'humeur, de l'épilepsie ; du syndrôme de Down ; de la chorée d'Huntington ; des désordres somatiques liés au stress ; des maladies neurodégénératives telles que la maladie de Pick, la maladie de Creutzfeldt-Jacob ; des désordres liés à la panique, à la phobie, au stress ;
- dans le traitement des modifications de la perméabilité de la barrière hématoencéphalique durant les processus inflammatoires et auto-immuns du système nerveux central, par exemple lors d'infections liées au SIDA ;
- comme myorelaxant et antispasmodique ;
- dans le traitement des nausées et vomissements aigus ou retardés et anticipés, par exemple les nausées et vomissements induits par des drogues comme les agents utilisés en chimiothérapie lors d'un cancer ; par thérapies par rayons lors d'irradiations du thorax ou de l'abdomen dans le traitement du cancer, de carcinoïdose ; par ingestion de poison ; par des toxines dues à des désordres métaboliques ou infectieux comme les gastrites, ou produites lors d'infection gastro-intestinale bactérienne ou virale ; lors d'une grossesse ; lors de troubles vestibulaires telles que le mal des transports, les vertiges, le syndrome de Ménière ; lors des maladies post-opératoires ; les nausées et vomissements induits par dialyse, par les prostaglandines ; par obstructions gastro-intestinales ; lors de motilité gastro-intestinale réduite ; lors de douleurs viscérales par infarctus du myocarde ou péritonite ; lors de migraine ; lors du mal d'altitude ; par ingestion d'analgésiques opiacés comme la morphine ; lors de reflux gastro-oesophagien ; lors d'indigestion acide ou de surconsommation de nourriture ou de boisson, lors d'acidité ou aigreur gastrique, régurgitation, brûlure d'estomac, par exemple épisodiques, nocturnes ou induites par un repas et dyspepsie ;
- dans le traitement des maladies du système gastro-intestinal telles que le syndrome du colon irritable, les ulcères gastriques et duodénaux, les ulcères oesophagiques, les diarrhées, les hypersécrétions, les lymphomes, les gastrites, les reflux gastro-oesophagiens, l'incontinence fécale, la maladie de Hirschsprung ;
- dans le traitement des maladies de la peau telles que le psoriasis, les prurits, les brûlures, notamment les coups de soleil ;
- dans le traitement des maladies du système cardiovasculaire telles que l'hypertension, les aspects vasculaires de la migraine, les oedèmes, la thrombose, l'angine de poitrine, les spasmes vasculaires, les maladies circulatoires dûes à une vasodilatation, la maladie de Raynaud, les fibroses, les maladies du collagène, l'athérosclérose ;
- dans le traitement des cancers du poumon à petites cellules ; des cancers du sein ; des tumeurs cérébrales ; des adéno-carcinomes de la sphère uro-génitale ; dans le traitement adjuvant pour prévenir les métastases ;
- les maladies de démyelination telles que la sclérose en plaques ou la sclérose latérale amyotrophique ;
- dans le traitement des maladies du système immunitaire liées à la suppression ou à la stimulation des fonctions des cellules immunes par exemple l'arthrite rhumatoïde, le psoriasis, la maladie de Crohn, le diabète, le lupus, les réactions de rejet après transplantation ;
- dans le traitement des troubles de la miction en particulier la pollakiurie ;
- dans le traitement de la réticulose histiocytaire comme dans les tissus lymphatiques ;
- comme anorexigène ;
- dans le traitement de l'emphysème ; du syndrome de Reiter ; des hémorroïdes ;

- dans le traitement des troubles oculaires telles que le glaucome, l'hypertension oculaire, le myosis, l'excès de larmes ;
- dans le traitement ou la prévention d'une attaque, de l'épilepsie, des traumatismes de la tête, des traumatismes du cordon spinal, des lésions ischémiques cérébrales dûe à une attaque ou à une occlusion vasculaire ;
- dans le traitement des troubles de la fréquence et du rythme cardiaque, en particulier ceux qui sont occasionnés par la douleur ou le stress ;
- dans le traitement des peaux sensibles et pour prévenir ou combattre les irritations de la peau ou des muqueuses, les pellicules, les érythèmes ou les prurits ;
- dans le traitement des troubles neurologiques de la peau tels que lichens, prurigos, toxidermies prurigineuses, prurits sévères d'origine neurogène ;
- dans le traitement des ulcères et de toutes maladies causées par Helicobacter Pylori ou une bactérie uréase positive gram négative ;
- dans le traitement des maladies causées par l'angiogénèse ou dont l'angiogénèse est un symtôme ;
- dans le traitement des algies oculaires et/ou palbébrales et/ou les dysesthésies oculaires ou palpébrales ;
- comme antiperspirant.

La présente invention inclut aussi une méthode pour traiter lesdites affections aux doses indiquées ci-dessus.
Les compositions pharmaceutiques selon la présente invention peuvent également renfermer d'autres produits actifs utiles pour traiter les maladies ou troubles indiqués ci-dessus, par exemple, des brochodilatateurs, des antitussifs, des antihistaminiques, des antiinflammatoires, des antiémétiques, des agents de chimiothérapie.
Les Préparations et Exemples suivants illustrent l'invention sans toutefois la limiter.
Dans les Préparations et dans les Exemples on utilise les abréviations suivantes :

DMF : diméthylformamide
DMSO : diméthylsulfoxyde
DCM : dichlorométhane
THF : tétrahydrofurane
éther chlorhydrique : solution saturée d'acide chlorhydrique dans l'éther
BOP : benzotriazol-1-yloxytris(diméthylamino)phosphonium hexafluorophosphate
F : point de fusion
TA : température ambiante
Teb : température d'ébullition
silice H : gel de silice 60H commercialisé par Merck (DARMSTAD)

Les spectres de résonance magnétique nucléaire du proton (RMN $^1H$) sont enregistrés à 200 MHz dans DMSO-$d_6$, en utilisant le pic du DMSO-$d_6$ comme référence. Les déplacements chimiques δ sont indiqués en parties par million (ppm). Les signaux observés sont exprimés ainsi :

s : singulet ; se : singulet élargi ; t : triplet ; qd : quadruplet ; m : massif : mt : multiplet.

PREPARATION 1.1 et 1.2

2-[2-(Benzoyloxy)éthyl]-2-(3,4-dichlorophényl)morpholine, isomère (-) (préparation 1.1) et 2-(3,4-dichlorophényl)-2-(2-hydroxyéthyl)morpholine, isomère (-) (préparation 1.2).

Préparation 1.1 : (II) :

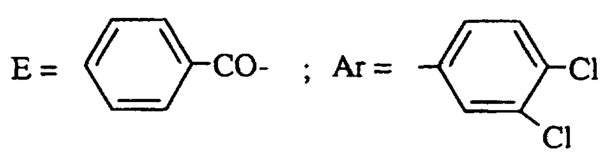

Préparation 1.2 : (II) : E = H ;

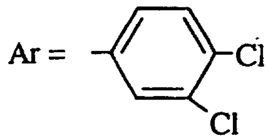

A) 2-(3,4-dichlorophényl)-2-hydroxyacétonitrile.

On laisse une nuit sous agitation à TA un mélange de 70 g de 3,4-dichlorobenzaldéhyde, 90 g de $Na_2S_2O_5$ dans 300 ml d'eau. On refroidit à 0°C le mélange réactionnel, ajoute goutte à goutte une solution de 52 g de KCN dans 100 ml d'eau et laisse sous agitation en laissant remonter la température à TA. On extrait le mélange réactionnel à l'éther, lave la phase organique à l'eau, sèche sur $Na_2SO_4$ et évapore sous vide le solvant. On obtient 76 g du produit attendu que l'on utilise tel quel.

B) 2-(3,4-dichlorophényl)-2-(tétrahydropyran-2-yloxy)acétonitrile.

On refroidit à 0°C une solution de 76 g du composé obtenu à l'étape précédente, 0,25 g d'acide *p*-toluènesulfonique monohydrate dans 300 ml de DCM, ajoute goutte à goutte une solution de 39 g de 3,4-dihydro-2*H*-pyrane dans 50 ml de DCM et laisse sous agitation en laissant remonter la température à TA. On lave le mélange réactionnel par une solution saturée de $NaHCO_3$, à l'eau, sèche la phase organique sur $Na_2SO_4$ et évapore sous vide le solvant. On obtient 33 g du produit attendu après cristallisation à 0°C dans le pentane, F = 61 °C.

C) 4-(Benzoyloxy)-2-(3,4-dichlorophényl)-2-(tétrahydropyran-2-yloxy)butanenitrile.

On refroidit à -60°C 56 ml d'une solution 2M de diisopropylamidure de lithium dans le THF, ajoute goutte à goutte une solution de 32 g du composé obtenu à l'étape précédente dans 50 ml de THF et laisse 1 heure sous agitation à -60°C. On ajoute ensuite à -60°C, goutte à goutte, une solution de 25,4 g de benzoate de 2-bromoéthyle dans 50 ml de THF et laisse sous agitation en laissant remonter la température à TA. On concentre sous vide le mélange réactionnel, extrait le résidu à l'éther, lave la phase organique à l'eau, par une solution tampon pH = 4, sèche sur $Na_2SO_4$ et évapore sous vide le solvant. On chromatographie le résidu sur silice en éluant par le mélange toluène/AcOEt (100/5 ; v/v). On obtient 34 g du produit attendu que l'on utilise tel quel.

D) 4-(Benzoyloxy)-2-(3,4-dichlorophényl)-2-(tétrahydropyran-2-yloxy)butylamine.

On hydrogène à TA et à pression atmosphérique un mélange de 34 g du composé obtenu à l'étape précédente, 10 g de nickel de Raney® dans 400 ml d'EtOH et 40 ml d'une solution concentrée d'ammoniaque. On filtre le catalyseur et concentre sous vide le filtrat. On reprend le résidu à l'eau, extrait à l'éther, lave la phase organique par une solution saturée de NaCl, sèche sur $Na_2SO_4$ et évapore sous vide le solvant. On chromatographie le résidu sur silice H en éluant par le gradient du mélange DCM/MeOH de (100/1 ; v/v) à (100/3 ; v/v). On obtient 16 g du produit attendu que l'on utilise tel quel.

E) Chlorhydrate de 4-(benzoyloxy)-2-(3,4-dichlorophényl)-2-hydroxybutylamine.

A une solution de 12 g du composé obtenu à l'étape précédente dans 50 ml de MeOH on ajoute à TA jusqu'à pH = 1 une solution saturée d'HCl gaz dans l'éther et laisse 1 heure sous agitation à TA. On concentre sous vide le mélange réactionnel, reprend le résidu au DCM, essore le précipité formé et le lave à l'éther. On obtient 3,4 g du produit attendu après recristallisation dans le propan-2-ol, F = 200-204°C.

F) 4-(Benzoyloxy)-2-(3,4-dichlorophényl)-2-hydroxybutylamine, isomère (-).

On chauffe à reflux une solution de 56,2 g d'acide (1S)-(+)-10-camphorsulfonique dans 660 ml de propan-2-ol, puis ajoute en une seule fois une solution de 78 g du composé obtenu à l'étape précédente, sous forme de base libre, dans 660 ml de propan-2-ol et laisse une nuit sous agitation en laissant revenir la température à TA. On essore les cristaux formés, les lave au propan-2-ol, puis à l'éther et les sèche sous vide. On obtient 115 g du sel d'acide camphorsulfonique. On recristallise le sel ainsi obtenu dans 3000 ml de propan-2-ol et obtient, après agitation une nuit à TA, essorage, lavage et séchage des cristaux formés, 100 g du sel d'acide camphorsulfonique. On recristallise à nouveau le sel ainsi obtenu dans 3000 ml d'EtOH 100 et obtient après, essorage, lavage et séchage des cristaux formés, 32 g du sel d'acide camphorsulfonique.

$\alpha_D^{20}$ = =-17,3° (c = 1 ; MeOH).

On reprend 30 g du sel ainsi obtenu par une solution de $Na_2CO_3$ à 10 %, extrait à l'AcOEt, lave la phase organique à l'eau jusqu'à pH neutre, sèche sur $Na_2SO_4$ et évapore sous vide le solvant. On obtient 17,72 g du produit attendu après séchage sous vide à 60°C, F = 101°C.
$\alpha_D^{20}$ = -49,1° (c = 1 ; MeOH).

G) N-(2-Chloroacétyl)-4-(benzoyloxy)-2-(3,4-dichlorophényl)-2-hydroxybutylamine, isomère (-).

On refroidit à 0°C une solution de 11,76 g du composé obtenu à l'étape précédente, 3,33 g de triéthylamine dans 150 ml de DCM, ajoute goutte à goutte une solution de 3,75 g de chlorure de chloroacétyle et laisse 5 minutes sous agitation. On concentre sous vide le mélange réactionnel, extrait le résidu à l'AcOEt, lave la phase organique à l'eau, par une solution tampon pH = 2, par une solution saturée de NaCl, sèche sur $Na_2SO_4$ et évapore sous vide le solvant. On obtient 14 g du produit attendu après cristallisation dans le mélange éther iso/pentane, F = 72-74°C.
$\alpha_D^{20}$ = -28° (c = 1 ; MeOH).

H) 6-[2-(Benzoyloxy)éthyl]-6-(3,4-dichlorophényl)morpholin-3-one, isomère (-).

On refroidit à -30°C une solution de 13,5 g de composé obtenu à l'étape précédente dans 400 ml de THF, ajoute en une fois 7,02 g de *tert*-butylate de potassium et laisse 20 minutes sous agitation à -30°C. On concentre sous vide à froid le mélange réactionnel, extrait le résidu à l'AcOEt, lave la phase organique par une solution tampon à pH = 2, à l'eau, par une solution saturée de NaCl, sèche sur $Na_2SO_4$ et évapore sous vide le solvant. On obtient 11,87 g du produit attendu après cristallisation dans le mélange éther iso/pentane, F = 134-137°C.
$\alpha_D^{20}$ = -4,9° (c = 1 ; MeOH).

I) 2-[2-(Benzoyloxy)éthyl]-2-(3,4-dichlorophényl)morpholine, isomère (-) (préparation 1.1) et 2-(3,4-dichlorophényl)-2-(2-hydroxyéthyl)morpholine, isomère (-) (préparation 1.2).

A 250 ml d'une solution 1M de borane dans le THF, on ajoute à TA et goutte à goutte une solution de 19,5 g du composé obtenu à l'étape précédente dans 100 ml de THF, puis chauffe à reflux pendant 3 heures. On ajoute ensuite goutte à goutte 120 ml de MeOH bouillant et continue le reflux pendant 30 minutes. On refroidit le mélange réactionnel au bain de glace, ajoute 50 ml d'une solution d'éther chlorhydrique et laisse une nuit sous agitation à TA. On concentre sous vide le mélange réactionnel, reprend le résidu par une solution de $Na_2CO_3$ à 10 %, extrait à l'AcOEt, lave la phase organique à l'eau, sèche sur $Na_2SO_4$ et évapore sous vide le solvant. On chromatographie le résidu sur gel de silice H en éluant par le gradient du mélange DCM/MeOH de (100/3 ; v/v) à (100/5 ; v/v). On sépare les deux composés :

- le moins polaire : composé de la préparation 1.1, m = 10,4 g, sous forme d'huile.
  $\alpha_D^{20}$ = -17° (c = 0,5 ; MeOH).
- le plus polaire : composé de la préparation 1.2, m = 5,3 g, sous forme d'huile.
  $\alpha_D^{20}$ = -20° (c = 0,5 ; MeOH).

PREPARATION 2.1

Acide 3,5-dichlorophénylacétique.

(III) : $R_1$ = Cl.

A) Chlorure de 3,5-dichlorobenzyle.

A une solution de 14,5 g d'alcool 3,5-dichlorobenzylique dans 150 ml de chloroforme, on ajoute goutte à goutte à TA une solution de 12,5 g de chlorure de thionyle dans 20 ml de chloroforme, puis chauffe à 40-50°C pendant 8 heures et laisse une nuit sous agitation à TA. On concentre sous vide et obtient 16 g du produit attendu que l'on utilise tel quel.

B) 3,5-Dichlorophénylacétonitrile.

A une solution de 16 g du composé obtenu à l'étape précédente dans 50 ml d'EtOH, on ajoute une solution de 6,5 g de cyanure de potassium dans 50 ml d'eau et chauffe à reflux pendant 4 heures. On concentre sous vide,

reprend le résidu à l'eau, extrait à l'éther, lave la phase organique à l'eau, sèche sur $Na_2SO_4$ et évapore sous vide le solvant. On chromatographie le résidu sur silice H en éluant par le mélange heptane/toluène (50/50 ; v/v) puis par du toluène. On obtient 7 g du produit attendu que l'on utilise tel quel.

C) Acide 3,5-dichlorophénylacétique.

A une solution de 7 g du composé obtenu à l'étape précédente dans 50 ml d'EtOH on ajoute une solution de 8,4 g de KOH dans 10 ml d'eau, puis chauffe à reflux pendant 5 heures. On concentre sous vide, reprend le résidu à l'eau, lave la phase aqueuse à l'éther, acidifie la phase aqueuse à pH = 1 par ajout d'HCl concentré et laisse une nuit sous agitation à TA. On essore le produit cristallise formé, le lave à l'eau et le sèche sous vide à 60°C. On obtient 7 g du produit attendu, F = 112-114,5°C.

PREPARATION 2.2

Acide 3,5-diéthylphénylacétique.

(III) : $R_1 = CH_2CH_3$.

A) 3,5-Diéthylbromobenzène.

On refroidit à -5°C un mélange de 20 g de 4-bromo-2,6-diéthylaniline, 160 ml d'acide acétique, 100 ml d'une solution d'HCl concentrée, 30 ml d'eau et 100 ml d'EtOH, ajoute goutte à goutte une solution de 6,6 g de nitrite de sodium dans 25 ml d'eau et laisse 30 minutes sous agitation à TA. On verse le mélange réactionnel sur 170 ml d'$H_3PO_2$ à 50 % refroidi à 0°C, laisse 2 heures sous agitation à 0°C puis 48 heures à TA. On extrait le mélange réactionnel à l'éther, lave la phase organique à l'eau, par une solution de NaOH 1N, à l'eau, sèche sur $Na_2SO_4$ et évapore sous vide le solvant. On chromatographie le résidu sur gel de silice en éluant au cyclohexane. On obtient 18 g du produit attendu.

B) 3,5-Diéthylbenzonitrile.

On chauffe pendant 15 heures à reflux un mélange de 24,7 g du composé obtenu à l'étape précédente et 12 g de cyanure cuivreux dans 70 ml de DMF. Après refroidissement à TA, on verse le mélange réactionnel sur 50 ml d'eau et laisse sous agitation à TA jusqu'à formation d'une gomme. On refroidit le mélange au bain de glace, ajoute 150 ml d'éthylènediamine et laisse 2 heures sous agitation à TA. On extrait le mélange à l'AcOEt, lave la phase organique à l'eau, sèche sur $Na_2SO_4$ et évapore sous vide le solvant. On chromatographie le résidu sur gel de silice en éluant par le mélange cyclohexane/AcOEt (95/5 ; v/v). On obtient 12 g du produit attendu.

C) Acide 3,5-diéthylbenzoïque.

A une solution de 12 g du composé obtenu à l'étape précédente dans 60 ml d'EtOH on ajoute une solution de 22 g de KOH dans 15 ml d'eau et chauffe à reflux pendant 24 heures. On concentre sous vide le mélange réactionnel, extrait le résidu à l'eau, lave la phase aqueuse à l'éther, acidifie la phase aqueuse à pH = 2 par ajout d'HCl concentrée, essore le précipité formé, le lave à l'eau et le sèche sous vide. On obtient 13 g du produit attendu.

D) Ester méthylique de l'acide 3,5-diéthylbenzoïque.

On chauffe à reflux pendant 48 heures un mélange de 13 g du composé obtenu à l'étape précédente dans 90 ml de MeOH et 10 gouttes d'$H_2SO_4$. On concentre sous vide le mélange réactionnel, reprend le résidu à l'eau, neutralise par ajout d'une solution à 10 % de $NaHCO_3$, extrait à l'éther, lave la phase organique par une solution de $NaHCO_3$ à 10 %, à l'eau, sèche sur $Na_2SO_4$ et évapore sous vide le solvant. On obtient 12 g du produit attendu.

E) Alcool 3,5-diéthylbenzylique.

On refroidit à 0°C une suspension de 2,5 g d'hydrure d'aluminium et de lithium dans 50 ml de THF, ajoute goutte à goutte une solution de 12 g du composé obtenu à l'étape précédente dans 50 ml de THF et laisse 30 minutes sous agitation. On hydrolyse le mélange réactionnel par ajout de 2,5 ml d'eau, 2,5 ml de NaOH 4N et 7,5 ml d'eau. On filtre les sels minéraux et concentre sous vide le filtrat. On obtient 10,9 g du produit attendu qui est utilisé tel quel.

F) Méthanesulfonate de 3,5-diéthylbenzyle.

A une solution de 10,9 g du composé obtenu à l'étape précédente et 7,4 g de triéthylamine dans 100 ml de DCM, on ajoute goutte à goutte, à TA, une solution de 8,4 g de chlorure de méthanesulfonyle dans 50 ml de DCM et laisse 30 minutes sous agitation. On concentre sous vide le mélange réactionnel, reprend le résidu à l'eau, extrait à l'éther, lave la phase organique à l'eau, sèche sur $Na_2SO_4$ et évapore sous vide le solvant. On obtient 16 g du produit attendu que l'on utilise tel quel.

G) 3,5-Diéthylphénylacétonitrile.

A une solution de 16 g du composé obtenu à l'étape précédente dans 100 ml de DMF, on ajoute une solution de 5,15 g de cyanure de potassium dans 20 ml d'eau et chauffe à 80°C pendant 1 heure. On concentre sous vide le mélange réactionnel, reprend le résidu à l'eau, extrait à l'éther, lave la phase organique à l'eau, sèche sur $Na_2SO_4$ et évapore sous vide le solvant. On chromatographie le résidu sur gel de silice H en éluant au DCM. On obtient 3 g du produit attendu.

H) Acide 3,5-diéthylphénylacétique.

A une solution de 3 g du composé obtenu à l'étape précédente dans 50 ml d'EtOH on ajoute une solution de 7,8 g de KOH dans 10 ml d'eau, puis chauffe à reflux pendant 5 heures. On concentre sous vide, reprend le résidu à l'eau, lave la phase aqueuse à l'éther, acidifie la phase aqueuse à pH = 1 par ajout d'HCl concentré et laisse une nuit sous agitation à TA. On essore le produit cristallisé formé, le lave à l'eau et le sèche sous vide. On obtient 2,5 g du produit attendu.

RMN $^1H$ : δ (ppm) : 1,1 : t : 6H ; 2,4 : qd : 4H ; 3,4 : s : 2H ; 6,8 : m : 3H ; 12,2 : se : 1H.

PREPARATION 2.3

Acide 3,5-diisopropylphénylacétique.

(III) : $R_1$=

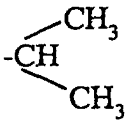

A) 4-Bromo-2,6-diisopropylamine.

On refroidit au bain de glace une solution de 17,7 g de 2,6-diisopropylamine dans 50 ml de MeOH et 10 ml d'acide acétique, ajoute, goutte à goutte et en maintenant une température inférieure à 15°C, une solution de 16 g de brome dans 50 ml d'acide acétique et laisse 2 heures sous agitation à TA. On concentre sous vide le mélange réactionnel, extrait le résidu à l'éther, lave plusieurs fois la phase organique à l'eau, sèche sur $Na_2SO_4$ et évapore sous vide le solvant. On obtient 25 g du produit attendu.

B) 3,5-Diisopropylbromobenzène.

On refroidit à 0°C un mélange de 25 g du composé obtenu à l'étape précédente dans 180 ml d'acide acétique, 120 ml d'eau et 35 ml d'une solution d'HCl concentrée, ajoute, goutte à goutte et en maintenant une température inférieure à 5°C, une solution de 7,6 g de nitrite de sodium dans 30 ml d'eau et laisse 30 minutes sous agitation à -5°C. On verse le mélange réactionnel sur 75 ml d'$H_3PO_2$ à 50 % refroidi à 0°C et laisse une nuit sous agitation en laissant remonter la température à TA. On extrait le mélange réactionnel à l'éther, lave la phase organique à l'eau, sèche sur $Na_2SO_4$ et évapore sous vide le solvant. On chromatographie le résidu sur gel de silice en éluant au cyclohexane. On obtient 16,2 g du produit attendu.

C) 3,5-Diisopropylbenzonitrile.

On chauffe pendant 18 heures à reflux un mélange de 16,2 g du composé obtenu à l'étape précédente et

6,95 g de cyanure cuivreux dans 50 ml de DMF. Après refroidissement à TA, on verse le mélange réactionnel sur 150 ml d'eau et laisse 30 minutes sous agitation à TA. On refroidit le mélange au bain de glace, ajoute 150 ml d'éthylènediamine et laisse 2 heures sous agitation à TA. On extrait le mélange à l'AcOEt, lave la phase organique à l'eau, sèche sur $Na_2SO_4$ et évapore sous vide le solvant. On chromatographie le résidu sur gel de silice en éluant par le mélange cyclohexane/AcOEt (100/5 ; v/v). On obtient 5,5 g du produit attendu.

D) Acide 3,5-diisopropylbenzoïque.

A une solution de 5,5 g du composé obtenu à l'étape précédente dans 50 ml d'EtOH, on ajoute une solution de 6,7 g de KOH dans 10 ml d'eau et chauffe à reflux pendant 18 heures. On concentre sous vide le mélange réactionnel, reprend le résidu à l'eau, lave la phase aqueuse à l'éther, acidifie la phase aqueuse à pH = 1 par ajout d'HCl concentré, essore le précipité formé, le lave à l'eau et le sèche. On obtient 5,4 g du produit attendu.

E) Ester éthylique de l'acide 3,5-diisopropylbenzoïque.

On chauffe à reflux pendant 18 heures un mélange de 5,4 g du composé obtenu à l'étape précédente dans 100 ml d'EtOH et 10 gouttes d'$H_2SO_4$. On concentre sous vide le mélange réactionnel, reprend le résidu à l'eau, neutralise par ajout d'une solution à 10 % de $NaHCO_3$, extrait à l'éther, lave la phase organique à l'eau, sèche sur $Na_2SO_4$ et évapore sous vide le solvant. On obtient 6 g du produit attendu.

F) Alcool 3,5-diisopropylbenzylique.

A une suspension de 1 g d'hydrure d'aluminium et de lithium dans 25 ml de THF, on ajoute, goutte à goutte et à TA, une solution de 6 g du composé obtenu à l'étape précédente dans 50 ml de THF et laisse 30 minutes sous agitation. On hydrolyse le mélange réactionnel par ajout de 1 ml d'eau, 1 ml de NaOH 4N puis 3 ml d'eau. On filtre les sels minéraux et concontre sous vide le filtrat. On extrait le résidu au DCM, lave la phase organique à l'eau, sèche sur $Na_2SO_4$ et évapore sous vide le solvant. On chromatographie le résidu sur gel de silice en éluant par le mélange cyclohexane/AcOEt (100/5 ; v/v). On obtient 4,4 g du produit attendu.

G) Méthanesulfonate de 3,5-diisopropylbenzyle.

A une solution de 4,4 g du composé obtenu à l'étape précédente et 2,5 g de triéthylamine dans 50 ml de DCM, on ajoute, goutte à goutte à TA, une solution de 2,88 g de chlorure de méthanesulfonyle dans 10 ml de DCM et laisse 30 minutes sous agitation. On lave le mélange réactionnel à l'eau, sèche la phase organique sur $Na_2SO_4$ et évapore sous vide le solvant. On obtient 6,2 g du produit attendu.

H) 3,5-Diisopropylphénylacétonitrile.

A une solution de 6,2 g du composé obtenu à l'étape précédente dans 40 ml d'EtOH, on ajoute une solution de 1,8 g de cyanure de potassium dans 10 ml d'eau et chauffe à reflux pendant 3 heures. On concentre sous vide le mélange réactionnel, reprend le résidu à l'eau, extrait à l'éther, lave la phase organique à l'eau, sèche sur $Na_2SO_4$ et évapore sous vide le solvant. On chromatographie le résidu sur gel de silice en éluant par le mélange cyclohexane/AcOEt (100/5 ; v/v). On obtient 2,2 g du produit attendu.

I) Acide 3,5-diisopropylphénylacétique.

A une solution de 2,2 g du composé obtenu à l'étape précédente dans 40 ml d'EtOH, on ajoute une solution de 3,8 g de KOH dans 10 ml d'eau et chauffe à reflux pendant 5 heures. On concentre sous vide le mélange réactionnel, reprend le résidu à l'eau, lave la phase aqueuse à l'éther, acidifie la phase aqueuse à pH = 1 par ajout d'HCl concentré, extrait au DCM, lave la phase organique à l'eau, sèche sur $Na_2SO_4$ et évapore sous vide le solvant. On obtient 2 g du produit attendu.

PREPARATION 3.1

Chlorhydrate de 2-(pipéridin-4-yl)isobutyramide.

$$(VII), HCl : X = -CH-C(CH_3)_2-CONH_2.$$

A) 2-Méthyl-2-(pyridin-4-yl)propionitrile.

On refroidit à 0°C un mélange de 3 g de chlorhydrate de pyridin-4-ylacétonitrile dans 50 ml de DMF, ajoute par petites portions 2,6 g d'hydrure de sodium à 60 % dans l'huile, et laisse 2 heures sous agitation à TA. On refroidit le mélange réactionnel au bain de glace, ajoute goutte à goutte 6 g d'iodure de méthyle et laisse une nuit sous agitation à TA. On verse le mélange réactionnel sur un mélange eau/glace, extrait à l'éther, lave la phase organique par une solution saturée de NaCl, sèche sur $MgSO_4$, filtre et évapore sous vide le solvant. On chromatographie le résidu sur gel de silice H en éluant au DCM puis par le mélange DCM/MeOH (98/2 ; v/v). On obtient 2,39 g du produit attendu sous forme d'huile qui cristallise.

B) Chlorhydrate de 2-(pyridin-4-yl)isobutyramide.

On chauffe à 100°C pendant 15 minutes un mélange de 2,39 g du composé obtenu à l'étape précédente et 10 ml d'une solution d'$H_2SO_4$ concentré. On refroidit le mélange réactionnel à TA, ajoute 50 g de glace, alcalinise à pH = 14 par ajout d'une solution de NaOH concentrée, filtre les sels minéraux, extrait le filtrat à l'AcOEt puis au DCM, sèche les phases organiques jointes sur $MgSO_4$, filtre et évapore sous vide les solvants (F = 134°C, base). On dissout le produit obtenu dans l'acétone, acidifie à pH = 1 par ajout d'éther chlorhydrique et essore le précipité formé. On obtient 2,9 g du produit attendu.

C) Chlorhydrate de 2-(pipéridin-4-yl)isobutyramide.

On hydrogène pendant 3 jours, à 60°C, sous une pression de 60 bars un mélange de 2,9 g du composé obtenu à l'étape précédente, 1 g de $PtO_2$ et 50 ml de MeOH. On filtre le catalyseur sur Célite®, lave au MeOH et concentre sous vide le filtrat. On reprend le résidu dans l'acétonitrile, essore le précipité formé, le lave à l'acétonitrile puis à l'éther. On obtient 2,5 g du produit attendu, F > 260°C.

PREPARATION 3.2

Dichlorhydrate de 2-(pipérazin-1-yl)isobutyramide.

$$(VII), 2HCl : X = -N-C(CH_3)_2-CONH_2.$$

A) 2-(4-Benzylpipérazin-1-yl)-2-méthylpropionitrile.

On mélange 4,5 ml d'acétone, 20 g de $MgSO_4$ sec, 10 g de N,N-diméthylacétamide, 10 g de 1-benzylpipérazine et 9,5 ml de 2-hydroxyisobutyronitrile et chauffe à 45°C pendant 48 heures sous forte agitation. On verse le mélange réactionnel sur de la glace et laisse 30 minutes sous agitation. On extrait le mélange à l'éther, lave plusieurs fois la phase organique à l'eau, sèche sur $Na_2SO_4$ et évapore sous vide le solvant. On obtient 13 g du produit attendu.

B) Dichlorhydrate de 2-(4-benzylpipérazin-1-yl)isobutyramide.

On chauffe rapidement à 110°C pendant 30 minutes un mélange de 13 g du composé obtenu à l'étape précédente et 130 ml d'une solution d'$H_2SO_4$ à 90 %. Après refroidissement à TA, on verse le mélange réactionnel sur de la glace, alcalinise à pH = 10 par ajout d'une solution de $NH_4OH$ concentrée et essore le produit cristallisé formé.

On dissout le produit dans du DCM, sèche la phase organique sur $MgSO_4$ et évapore sous vide le solvant. On reprend le produit dans l'éther chlorhydrique et essore le précipité formé. On obtient 9,5 g du produit attendu.

C) Dichlorhydrate de 2-(pipérazin-1-yl)isobutyramide.

On hydrogène pendant une nuit, à TA et à pression atmosphérique, un mélange de 1,3 g du composé obtenu à l'étape précédente et 0,18 g de Palladium sur charbon à 10 % dans 30 ml d'EtOH 95. On filtre le catalyseur sur Célite® et concentre sous vide le filtrat. On obtient 0,6 g du produit attendu.

PREPARATION 3.3

Dichlorhydrate de 1-(pipérazin-1-yl)cyclohexanecarboxamide.

(VII), 2HCl : X = 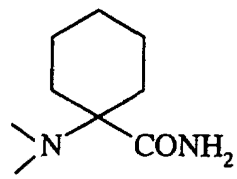

A) 1-(4-Benzylpipérazin-1-yl)cyclohexanecarbonitrile.

On mélange 5,7 g de cyclohexanone, 20 g de $MgSO_4$ sec, 10 g de N,N-diméthylacétamide, 10 g de 1-benzylpipérazine et 9,5 ml de 2-hydroxyisobutyronitrile et chauffe à 45°C pendant 48 heures sous forte agitation. On verse le mélange réactionnel sur de la glace et laisse 30 minutes sous agitation. On extrait le mélange à l'éther, lave plusieurs fois la phase organique à l'eau, sèche sur $Na_2SO_4$ et évapore sous vide le solvant. On obtient 15 g du produit attendu.

B) Dichlorhydrate de 1-(4-benzylpipérazin-1-yl)cyclohexanecarboxamide.

On prépare ce composé selon le mode opératoire décrit à l'étape B de la Préparation 3.2 à partir de 15 g du composé obtenu à l'étape précédente et 50 ml d'une solution d'$H_2SO_4$ à 90 %. On obtient 5,5 g du produit attendu.

C) Dichlorhydrate de 1-(pipérazin-1-yl)cyclohexanecarboxamide.

On prépare ce composé selon le mode opératoire décrit à l'étape C de la Préparation 3.2 à partir de 2,3 g du composé obtenu à l'étape précédente, 0,3 g de palladium sur charbon à 10 % dans 30 ml d'EtOH 95. On obtient 1,6 g du produit attendu.

PREPARATION 3.4

Diformate de N,N-diméthyl-2-(pipérazin-1-yl)isobutyramide.

(VII), $2HCO_2H$ :

$$X = -N-C(CH_3)_2-CON(CH_3)_2$$

A) N,N-diméthyl-2-(4-benzylpipérazin-1-yl)isobutyramide.

A un mélange de 2,6 g de composé obtenu à l'étape B de la Préparation 3.2 (base libre) dans 50 ml de THF anhydre, on ajoute par portions 1,44 g d'hydrure de sodium à 60 % dans l'huile. On ajoute ensuite, en goutte à goutte, 1,3 ml d'iodure de méthyle et laisse 4 heures sous agitation à TA. On verse le mélange réactionnel dans l'eau, extrait à l'éther, sèche la phase organique sur $MgSO_4$ et évapore sous vide les solvants. On obtient 1,8 g du produit attendu.

B) Diformate de N,N-diméthyl-2-(pipérazin-1-yl)isobutyramide.

A une solution de 1,8 g du composé obtenu à l'étape précédente dans 30 ml de MeOH, on ajoute 2 g de formiate d'ammonium, 0,5 g de palladium sur charbon à 5 % et laisse 4 heures sous agitation à TA. On filtre le catalyseur sur Célite® et concentre sous vide le filtrat. On reprend le résidu dans l'AcOEt, essore le précipité formé, le lave à l'AcOEt et le sèche. On obtient 1,2 g du produit attendu.

PREPARATION 3.5

Chlorhydrate de 1-(pipéridin-4-yl)cyclohexanecarboxamide.

(VII), HCl :

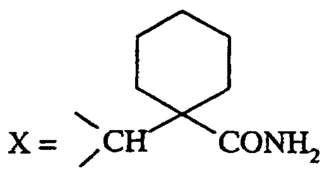

A) 1-(Pyridin-4-yl)cyclohexanecarbonitrile.

On refroidit à 0°C un mélange de 3 g de chlorhydrate de pyridin-4-ylacétonitrile dans 50 ml de DMF, ajoute par petites portions 2,6 g d'hydrure de sodium à 60 % dans l'huile et laisse 1 heure 30 minutes sous agitation à TA. On refroidit le mélange réactionnel au bain de glace, ajoute goutte à goutte 2,7 ml de 1,5-dibromopentane et laisse 48 heures sous agitation à TA. On verse le mélange réactionnel sur une solution saturée de $NH_4Cl$, extrait à l'éther, lave trois fois la phase organique à l'eau, sèche sur $MgSO_4$ et évapore sous vide le solvant. On chromatographie le résidu sur gel de silice H en éluant au DCM puis par le mélange DCM/MeOH (98/2 ; v/v). On obtient 2,5 g du produit attendu, F = 79°C.

B) Chlorhydrate de 1-(pyridin-4-yl)cyclohexanecarboxamide.

On chauffe à 100°C pendant 15 minutes un mélange de 2,5 g du composé obtenu à l'étape précédente et 15 ml d'une solution d'$H_2SO_4$ concentrée. On refroidit le mélange réactionnel à TA, le verse sur de la glace, alcalinise à pH = 14 par ajout d'une solution de NaOH concentrée, essore le précipité formé, le lave à l'eau et le sèche. On dissout le produit obtenu dans l'acétone, acidifie à pH = 1 par ajout d'éther chlorhydrique, laisse 30 minutes sous agitation à TA et essore le précipité formé. On obtient 3 g du produit attendu, F = 224°C (déc.).

C) Chlorhydrate de 1-(pipéridin-4-yl)cyclohexanecarboxamide.

On hydrogène pendant 3 jours, à 60°C, sous une pression de 80 bars, un mélange de 2,9 g du composé obtenu à l'étape précédente, 0,5 g de $PtO_2$ et 50 ml de MeOH. On filtre le catalyseur sur Célite®, et concentre sous vide le filtrat. On reprend le résidu dans l'acétonitrile, laisse 1 heure sous agitation à TA, et essore le précipité formé. On obtient 2,7 g du produit attendu, F = 235°C.

PREPARATION 3.6

Chlorhydrate de 1-(pipéridin-4-yl)cyclopropane carboxamide.

(VII), HCl :

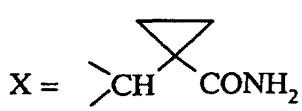

A) 1-(Pyridin-4-yl)cyclopropanecarbonitrile.

A un mélange de 2,5 g d'amidure de sodium dans 80 ml de DCM, on ajoute 3,5 g de pyridin-4-ylacétonitrile puis 2,6 ml de 1,2-dibromoéthane et laisse une nuit sous agitation à TA. On verse le mélange réactionnel dans l'eau, extrait à l'AcOEt, lave la phase organique à l'eau, sèche sur $Na_2SO_4$ et évapore sous vide les solvants. On chromatographie le résidu sur gel de silice en éluant au DCM puis par le mélange DCM/MeOH de (99/1 ; v/v) à (95/5 ; v/v). On obtient 2,5 g du produit attendu.

B) Chlorhydrate de 1-(pyridin-4-yl)cyclopropanecarboxamide.

On chauffe rapidement à 100°C un mélange de 2,5 g du composé obtenu à l'étape précédente et 20 ml d'une solution d'$H_2SO_4$ à 96 % et laisse 1 heure sous agitation à 100°C. Après refroidissement à TA, on verse le mélange réactionnel sur de la glace, neutralise à pH = 7 par ajout d'une solution de $NH_4OH$ à 20 %, essore le précipité formé, le lave à l'eau et le sèche. On dissout le précipité dans le DCM, acidifie à pH = 1 par ajout d'éther chlorhydrique et essore le précipité formé. On obtient 1,8 g du produit attendu.

C) Chlorhydrate de 1-(pipéridin-4-yl)cyclopropane carboxamide.

On hydrogène pendant 15 heures, à 80°C et sous une pression de 100 bars, un mélange de 1,8 g du composé obtenu à l'étape précédente et 0,6 g de $PtO_2$ dans 50 ml de MeOH. On filtre le catalyseur sur Célite®, concentre sous vide le filtrat jusqu'à un volume de 5 ml, et ajoute de l'acétonitrile jusqu'à cristallisation. On obtient 1,7 g du produit attendu après essorage puis séchage.

PREPARATION 3.7

Chlorhydrate de N,N-diméthyl-2-(pipéridin-4-yl)isobutyramide.

$$(VII), HCl : X = -\underset{|}{C}H-C(CH_3)_2-CON(CH_3)_2$$

A) Ester éthylique de l'acide 1-benzylpipéridine-4-carboxylique.

A un mélange de 25 g d'isonipécotate d'éthyle et 25 g de $K_2CO_3$ dans 125 ml de DMF, on ajoute goutte à goutte 30 g de bromure de benzyle en maintenant la température du mélange réactionnel entre 25 et 30°C, puis laisse 1 heure sous agitation à TA. On verse le mélange réactionnel sur 1 litre d'eau glacée, extrait deux fois à l'éther, lave la phase organique à l'eau, sèche sur $MgSO_4$ et évapore sous vide le solvant. On distille l'huile résultante obtenue sous pression réduite. On obtient 29,2 g du produit attendu, Teb = 120-122°C sous 2,7 Pa.

B) 2-(1-Benzylpipéridin-4-yl)propan-2-ol.

A 200 ml d'une solution 1,5 M de méthyllithium, comme complexe avec du bromure de lithium, dans l'éther, on ajoute, sous atmosphère d'argon, en goutte à goutte et en maintenant la température du milieu entre 25 et 30°C, une solution de 24,73 g du composé obtenu à l'étape précédente dans 100 ml de benzène, puis chauffe à reflux pendant 48 heures. On refroidit le mélange réactionnel à TA, puis le verse sur 400 ml d'une solution saturée de $NH_4Cl$ dans l'eau préalablement refroidie au bain de glace. On extrait le mélange trois fois à l'éther, sèche les phases organiques jointes sur $MgSO_4$ et concentre sous vide le solvant. On dissout le résidu dans 100 ml d'acétone, refroidit à 10°C, acidifie à pH = 1 par ajout d'éther chlorhydrique, essore le précipité formé et le lave par un mélange acétone/éther (50/50 ; v/v). On obtient 24,5 g du produit attendu sous forme de chlorhydrate, F = 204°C. Pour libérer la base, on reprend le chlorhydrate dans une solution concentrée de NaOH, extrait à l'éther, sèche sur $MgSO_4$ et évapore sous vide le solvant. On obtient 21 g du produit attendu, F = 66°C.

C) Acide 2-(1-benzylpipéridin-4-yl)-2-méthylpropionique.

On refroidit à 3°C un mélange de 5,98 g d'acide sulfurique à 95 % et 4,42 g d'acide sulfurique fumant à 30 % en $SO_3$, ajoute, goutte à goutte et en maintenant la température en dessous de 10°C, une solution de 2 g du composé

obtenu à l'étape précédente dans 1,55 g d'acide formique à 100 %. On laisse 2 heures sous agitation à 3-5°C, puis laisse remonter la température à TA et abandonne une nuit à TA. On verse le mélange réactionnel sur de la glace, amène à pH = 6,5 par ajout d'une solution concentrée de NaOH et par ajout d'une solution concentrée de $NH_4OH$, extrait trois fois au DCM, sèche les phases organiques jointes sur $MgSO_4$ et concentre sous vide le solvant. On reprend le résidu à l'acétone, essore le précipité et le sèche. On obtient 1,22 g du produit attendu, F = 195°C.

D) Chlorhydrate de N,N-diméthyl-2-(1-benzylpipéridin-4-yl)isobutyramide.

On laisse 1 heure sous agitation à TA un mélange de 1,2 g du composé obtenu à l'étape précédente, 0,8 ml de triéthylamine, 2,8 ml d'une solution 2 M de diméthylamine dans le THF et 2,5 g de BOP dans 20 ml de DCM. On concentre sous vide le mélange réactionnel, reprend le résidu à l'éther, lave la phase organique à l'eau, par une solution de NaOH 1N, par une solution saturée de NaCl, sèche sur $MgSO_4$ et concentre sous vide le solvant. On chromatographie le résidu sur gel de silice H en éluant au DCM, puis par le gradient du mélange DCM/MeOH de (99/1 ; v/v) à (95/5 ; v/v). On dissout le produit obtenu dans l'acétone, acidifie à pH = 1 par ajout d'éther chlorhydrique, essore le précipité formé et le sèche. On obtient 0,8 g du produit attendu, F = 229°C.

E) Chlorhydrate de N,N-diméthyl-2-(pipéridin-4-yl)isobutyramide.

On hydrogène pendant une nuit, sous pression atmosphérique et à TA, un mélange de 0,8 g du composé obtenu à l'étape précédente et 0,2 g de palladium sur charbon à 10 % dans 20 ml de MeOH. On filtre le catalyseur sur Célite® et concentre sous vide le filtrat. On dissout le résidu dans l'acétonitrile, ajoute de l'éther, essore le précipité formé et le sèche. On obtient 0,51 g du produit attendu, F = 258°C.

PREPARATION 3.8

Chlorhydrate de 2-méthyl-1-(morpholin-4-yl)-2-(pipéridin-4-yl)propan-1-one.

(VII), HCl : X = 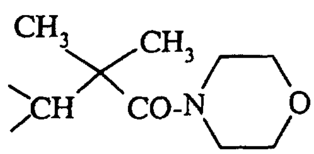

A) Chlorhydrate de 2-(1-benzylpipéridin-4-yl)-2-méthyl-1-(morpholin-4-yl)propan-1-one.

On chauffe à 80°C pendant 3 heures un mélange de 1 g du composé obtenu à l'étape C de la Préparation 3.7 et 1,2 ml de chlorure de thionyle dans 20 ml de 1,2-dichloroéthane. On concentre sous vide le mélange réactionnel, dissout le chlorure d'acide ainsi obtenu dans 20 ml de DCM, ajoute cette solution à un mélange de 0,7 g de morpholine, 1,6 ml de triéthylamine dans 20 ml de DCM préalablement refroidi à 0°C et laisse 24 heures sous agitation à TA. On concentre sous vide le mélange réactionnel, extrait le résidu à l'éther, lave la phase organique par une solution de NaOH 1N, à l'eau, sèche sur $MgSO_4$ et évapore sous vide le solvant. On dissout le produit obtenu dans l'acétone, acidifie à pH = 1 par ajout d'éther chlorhydrique, essore le précipité formé et le sèche. On obtient 0,7 g du produit attendu.

B) Chlorhydrate de 2-méthyl-1-(morpholin-4-yl)-2-(pipéridin-4-yl)propan-1-one.

On laisse 4 heures sous agitation à TA un mélange de 0,7 g du composé obtenu à l'étape précédente, 0,7 g de formiate d'ammonium et 0,2 g de palladium sur charbon à 10 % dans 10 ml de MeOH. On filtre le catalyseur sur Célite® et concentre sous vide le filtrat. On dissout le résidu dans l'acétonitrile, ajoute de l'éther, essore le précipité formé et le sèche. On obtient 0,46 g du produit attendu, F = 225°C.

EXEMPLE 1

Dichlorohydrate de 2-[2-(4-cyclohexylpipérazin-1-yl)éthyl]-2-(3,4-dichloro phényl)-4-[2-(3,5-diméthylphényl)acétyl] morpholine, monohydrate, isomère (-).

(I), 2HCl : X = 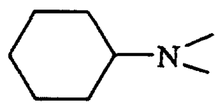 ; $R_1 = -CH_3$ ; Ar = 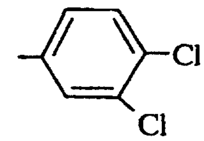

A) 2-[2-(Benzoyloxy)éthyl]-2-(3,4-dichlorophényl)-4-[2-(3,5-diméthylphényl)acétyl] morpholine, isomère unique.

On refroidit au bain de glace un mélange de 2,2 g du composé obtenu à la Préparation 1.1, 1,48 g de triéthylamine et 0,96 g d'acide 3,5-diméthylphénylacétique dans 40 ml de DCM, ajoute 2,85 g de BOP et laisse 3 heures sous agitation en laissant remonter la température à TA. On concentre sous vide le mélange réactionnel, extrait le résidu à l'AcOEt, lave la phase organique à l'eau, par une solution de $Na_2CO_3$ à 10 %, à l'eau, par une solution saturée de NaCl, sèche sur $Na_2SO_4$ et évapore sous vide le solvant. On chromatographie le résidu sur gel de silice H en éluant par le mélange DCM/MeOH (100/0,5 ; v/v). On obtient 2,4 g du produit attendu sous forme d'huile.

B) 2-(3,4-Dichlorophényl)-2-(2-hydroxyéthyl)-4-[2-(3,5-diméthylphényl)acétyl] morpholine, isomère unique.

On laisse 30 minutes sous agitation à TA un mélange de 2,4 g du composé obtenu à l'étape précédente et 1,7 ml d'une solution aqueuse de NaOH à 30 %, dans 30 ml de MeOH. On concentre sous vide le mélange réactionnel, reprend le résidu à l'eau, extrait à l'AcOEt, lave la phase organique à l'eau, par une solution saturée de NaCl, sèche sur $Na_2SO_4$ et évapore sous vide le solvant. On obtient 1,92 g du produit attendu sous forme d'huile.

C) 2-(3,4-Dichlorophényl)-2-[2-(méthanesulfonyloxy)éthyl]-4-[2-(3,5-diméthyl phényl)acétyl]morpholine, isomère unique.

On refroidit au bain de glace une solution de 1,92 g du composé obtenu à l'étape précédente et 0,94 ml de triéthylamine dans 30 ml de DCM, ajoute goutte à goutte une solution de 0,57 g de chlorure de méthanesulfonyle dans 10 ml de DCM et laisse 5 minutes sous agitation. On concentre sous vide le mélange réactionnel, reprend le résidu à l'eau, extrait à l'AcOEt, lave la phase organique à l'eau, par une solution saturée de NaCl, sèche sur $Na_2SO_4$ et évapore sous vide le solvant. On obtient 2,22 g du produit attendu sous forme d'huile.

D) Dichlorohydrate de 2-[2-(4-cyclohexylpipérazin-1-yl)éthyl]-2-(3,4-dichloro phényl)-4-[2-(3,5-diméthylphényl)acétyl] morpholine, monohydrate, isomère (-).

On chauffe à 80°C pendant 3 heures un mélange de 1,1 g du composé obtenu à l'étape précédente, 0,56 g de 1-cyclohexylpipérazine et 0,61 g de $K_2CO_3$ dans 2 ml de DMF. Après refroidissement à TA, on ajoute de l'eau glacée au mélange réactionnel, extrait à l'AcOEt, lave la phase organique à l'eau, par une solution saturée de NaCl, sèche sur $Na_2SO_4$ et évapore sous vide le solvant. On chromatographie le résidu sur gel de silice H en éluant par le mélange DCM/MeOH (100/3 ; v/v). On dissout le produit obtenu dans DCM, ajoute de l'éther chlorhydrique jusqu'à pH = 1 et concentre sous vide. On obtient 0,51 g du produit attendu après concrétisation dans le mélange DCM/pentane.
$\alpha_D^{20}$ = -28,7° (c = 1 ; MeOH).

RMN $^1H$ : $\delta$ (ppm) : 0,7 à 2,45 : m : 18H ; 2,5 à 4,65 : m : 19H ; 6,4 à 7,8 : m : 6H ; 11,8 : s : 2H.

EXEMPLE 2

Dichlorohydrate de 2-[2-(4-cyclohexylpipérazin-1-yl)éthyl]-2-(3,4-dichloro phényl)-4-[2-(3,5-dichlorophényl)acétyl] morpholine, hémihydrate, isomère (+).

(I), 2HCl : X = 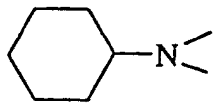 ; $R_1$ = Cl ; Ar = 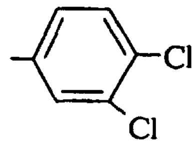

A) 2-[2-(Benzoyloxy)éthyl]-2-(3,4-dichlorophényl)-4-[2-(3,5-dichlorophényl)acétyl] morpholine, isomère unique.

On prépare ce composé selon le mode opératoire décrit à l'étape A de l'EXEMPLE 1 à partir de 1,92 g du composé obtenu à la Préparation 1.1, 1,27 g de triéthylamine, 1,04 g d'acide 3,5-dichlorophénylacétique, 35 ml de DCM et 2,46 g de BOP. On obtient 2,2 g du produit attendu sous forme d'huile.

B) 2-(3,4-Dichlorophényl)-4-[2-(3,5-dichlorophényl)acétyl]-2-(2-hydroxyéthyl) morpholine, isomère unique.

On prépare ce composé selon le mode opératoire décrit à l'étape B de l'EXEMPLE 1 à partir de 2,2 g du composé obtenu à l'étape précédente, 1,5 ml d'une solution aqueuse de NaOH à 30 % et 30 ml de MeOH. On obtient 1,8 g du produit attendu sous forme d'huile.

C) 2-(3,4-dichlorophényl)-4-[2-(3,5-dichlorophényl)acétyl]-2-[2-(méthane sulfonyloxy)éthyl]morpholine, isomère unique.

On prépare ce composé selon le mode opératoire décrit à l'étape C de l'EXEMPLE 1 à partir de 1,8 g du composé obtenu à l'étape précédente, 0,59 g de triéthylamine, 30 ml de DCM et 0,49 g de chlorure de méthanesulfonyle dans 10 ml de DCM. On obtient 2 g du produit attendu sous forme d'huile.

D) Dichlorohydrate de 2-[2-(4-cyclohexylpipérazin-1-yl)éthyl]-2-(3,4-dichloro phényl)-4-[2-(3,5-dichlorophényl)acétyl] morpholine, hémihydrate, isomère (+).

On prépare ce composé selon le mode opératoire décrit à l'étape D de l'EXEMPLE 1 à partir de 1 g du composé obtenu à l'étape précédente, 0,45 g de 1-cyclohexylpipérazine, 0,51 g de $K_2CO_3$ et 2 ml de DMF. On obtient 0,54 g du produit attendu.
$\alpha_D^{20}$ = +1,2° (c = 1 ; MeOH).
RMN $^1H$ : δ (ppm) : 0,8 à 2,5 : m : 12H ; 2,55 à 4,4 : m : 19H ; 7,0 à 8,0 : m : 6H; 11,6: s : 2H.

EXEMPLE 3

Dichlorohydrate de 2-[2-(4-cyclohexylpipérazin-1-yl)éthyl]-2-(3,4-dichloro phényl)-4-[2-[3,5-bis(trifluorométhyl)phényl] acétyl]morpholine, hémihydrate, isomère (+).

(I), 2HCl : X = 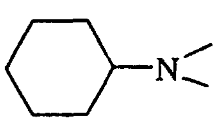 ; $R_1$ = $CF_3$ ; Ar = 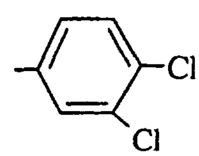

A) 2-[2-(Benzoyloxy)éthyl]-2-(3,4-dichlorophényl)-4-[2-[3,5-bis(trifluorométhyl) phényl]acétyl]morpholine, isomère unique.

On prépare ce composé selon le mode opératoire décrit à l'étape A de l'EXEMPLE 1, à partir de 2,23 g du composé obtenu à la Préparation 1.1, 1,48 g de triéthylamine, 1,59 g d'acide 3,5-bis(trifluorométhyl)phénylacétique, 40 ml de DCM et 2,85 g de BOP. On obtient 2,4 g du produit attendu sous forme d'huile.

B) 2-(3,4-Dichlorophényl)-4-[2-[3,5-bis(trifluorométhyl)phényl]acétyl]-2-(2-hydroxyéthyl)morpholine, isomère unique.

On prépare ce composé selon le mode opératoire décrit à l'étape B de l'EXEMPLE 1 à partir de 2,4 g du composé obtenu à l'étape précédente, 1,4 ml d'une solution aqueuse de NaOH à 30 % et 30 ml de MeOH. On obtient 2 g du produit attendu sous forme d'huile.

C) 2-(3,4-Dichlorophényl)-4-[2-[3,5-bis(trifluorométhyl)phényl]acétyl]-2-[2-(méthane sulfonyloxy)éthyl]morpholine, isomère unique.

On prépare ce composé selon le mode opératoire décrit à l'étape C de l'EXEMPLE 1 à partir de 2 g du composé obtenu à l'étape précédente, 0,57 g de triéthylamine, 30 ml de DCM et 0,47 g de chlorure de méthanesulfonyle dans 10 ml de DCM. On obtient 2,29 g du produit attendu sous forme d'huile.

D) Dichlorohydrate de 2-[2-(4-cyclohexylpipérazin-1-yl)éthyl]-2-(3,4-dichloro phényl)-4-[2-[3,5-bis(trifluorométhyl)phényl]acétyl]morpholine, hémihydrate, isomère (+).

On chauffe à 80-100°C pendant 2 heures un mélange de 1 g du composé obtenu à l'étape précédente, 0,29 g de 1-cyclohexylpipérazine et 0,71 g de $K_2CO_3$ dans 3 ml de DMF. Après refroidissement à TA, on verse le mélange réactionnel dans l'eau, extrait à l'AcOEt, lave la phase organique à l'eau, sèche sur $Na_2SO_4$ et évapore sous vide le solvant. On chromatographie le résidu sur gel de silice H en éluant par le gradient du mélange DCM/MeOH de (100/2 ; v/v) à (100/5 ; v/v). On dissout le produit obtenu dans du DCM, ajoute de l'éther chlorhydrique jusqu'à pH = 1 et concentre sous vide. On obtient 0,6 g du produit attendu après concrétisation dans le mélange DCM/pentane.
$\alpha_D^{20}$ = +23,4° (c = 0,5 ; MeOH).

RMN $^1H$ : δ (ppm) : 0,8 à 2,6 : m : 12H ; 2,6 à 4,3 : m : 19H ; 7,1 à 8,0 : m : 6H ; 11,8 : m : 2H.

EXEMPLE 4

Chlorhydrate de 2-[2-[4-(1-carbamoyl-1-méthyléthyl)pipéridin-1-yl]éthyl]-2-(3,4-dichlorophényl)-4-[2-(3,5-diméthylphényl)acétyl]morpholine, sesquihydrate, isomère (-).

(I), HCl : X = $H_2NCO-C(CH_3)_2-CH-$ ; $R_1 = -CH_3$ ; Ar = 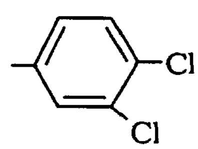

A) 2-(3,4-Dichlorophényl)-2-(formylméthyl)-4-[2-(3,5-diméthylphényl)acétyl] morpholine, isomère unique.

On refroidit à -60°C, sous atmosphère d'azote, une solution de 0,63 ml de chlorure d'oxalyle dans 20 ml de DCM, ajoute goutte à goutte une solution de 1,3 ml de DMSO dans 20 ml de DCM, puis une solution de 2,55 g du composé obtenu à l'étape B de l'Exemple 1 et 1,84 ml de DMSO dans 20 ml de DCM. On laisse 30 minutes sous agitation à -60°C, remonte à -50°C, ajoute 5,2 ml de triéthylamine et laisse sous agitation en laissant remonter la température à TA. On lave le mélange réactionnel par une solution d'HCl 2N, à l'eau, par une solution saturée de $NaHCO_3$, à l'eau, sèche la phase organique sur $MgSO_4$ et évapore sous vide le solvant. On obtient 2,38 g du produit attendu.

B) Chlorhydrate de 2-[2-[4-(1-carbamoyl-1-méthyléthyl)pipéridin-1-yl]éthyl]-2-(3,4-dichlorophényl)-4-[2-(3,5-diméthylphényl)acétyl]morpholine, sesquihydrate, isomère (-).

A une solution de 0,64 g du composé obtenu à l'étape précédente dans 30 ml de DCM, on ajoute à TA et sous atmosphère d'azote, 0,26 g du composé obtenu à la Préparation 3.1 (base libre), puis 0,74 g de triacétoxyborohydrure de sodium et 8 gouttes d'acide acétique et laisse une nuit sous agitation à TA. On alcalinise le mélange réactionnel à pH = 8 par ajout d'une solution saturée de $NaHCO_3$, extrait au DCM, lave trois fois la phase organique à l'eau, sèche sur $MgSO_4$ et évapore sous vide le solvant. On chromatrographie le résidu sur gel de silice H en éluant par le gradient du mélange DCM/MeOH de (100/1 ; v/v) jusqu'à (100/5 ; v/v). On dissout le produit obtenu dans du DCM, ajoute de l'éther chlorhydrique jusqu'à pH = 1, essore le précipité formé et le sèche. On obtient 0,631 g du produit attendu.

$\alpha_D^{20}$ = -23,8° (c = 0,5 ; MeOH).

RMN $^1H$ : δ (ppm) : 0,8 à 1,2 : se : 6H ; 1,2 à 2,0 : m : 6H ; 2,0 à 4,8 : m : 21H ; 6,6 à 8,8 : m : 8H ; 10,2 : se : 1H.

EXEMPLE 5

Chlorhydrate de 2-[2-[4-(1-carbamoyl-1-méthyléthyl)pipéridin-1-yl]éthyl]-2-(3,4-dichlorophényl)-4-[2-(3,5-diéthylphényl)acétyl]morpholine, hémihydrate, isomère (-).

(I), HCl : X = $H_2NCO-C(CH_3)_2-CH-$ ; $R_1 = -CH_2CH_3$ ; Ar = 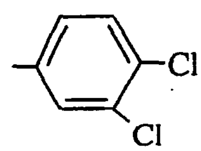

A) 2-[2-(Benzoyloxy)éthyl]-2-(3,4-dichlorophényl)-4-[2-(3,5-diéthylphényl)acétyl] morpholine, isomère unique.

On refroidit à 0°C une solution de 2,27 g du composé obtenu à la Préparation 1.1 dans 25 ml de DCM, ajoute 1,15 g du composé obtenu à la Préparation 2.2, 0,72 g de triéthylamine puis 3,17 g de BOP et laisse 1 heure sous agitation. On extrait le mélange réactionnel au DCM, lave la phase organique à l'eau, par une solution tampon pH = 2, à l'eau, par une solution de $Na_2CO_3$ à 10 %, à l'eau, sèche sur $Na_2SO_4$ et évapore sous vide le solvant. On chromatographie le résidu sur gel de silice H en éluant au DCM puis par le mélange DCM/MeOH (100/1 ; v/v). On obtient 3,1 g du produit attendu.

B) 2-(3,4-Dichlorophényl)-2-(2-hydroxyéthyl)-4-[2-(3,5,diéthylphényl)acétyl] morpholine, isomère unique.

On laisse 1 heure sous agitation à TA un mélange de 3,1 g du composé obtenu à l'étape précédente et 1,5 ml d'une solution aqueuse de NaOH à 30 %, dans 130 ml de MeOH. On concentre sous vide le mélange réactionnel, extrait le résidu par un mélange AcOEt/éther (50/50 ; v/v), lave trois fois la phase organique à l'eau, sèche sur $Na_2SO_4$ et évapore sous vide le solvant. On chromatographie le résidu sur gel de silice H en éluant au DCM puis par le mélange DCM/MeOH (100/2 ; v/v). On obtient 2 g du produit attendu.

C) 2-(3,4-Dichlorophényl)-2-(formylméthyl)-4-[2-(3,5-diéthylphényl)acétyl] morpholine, isomère unique.

On prépare ce composé selon le mode opératoire décrit à l'étape A de l'Exemple 4 à partir de 0,67 g de chlorure d'oxalyle dans 20 ml de DCM, 1,0 g de DMSO dans 10 ml de DCM, 2,0 g du composé obtenu à l'étape précédente et 1,44 g de DMSO dans 20 ml de DCM et 2,9 g de triéthylamine. On obtient 1,95 g du produit attendu.

D)Chlorhydratede2-[2-[4-(1-carbamoyl-1-méthyléthyl)pipéridin-1-yl]éthyl]-2-(3,4-dichlorophényl)-4-[2-(3,5-diéthylphényl)acétyl]morpholine, hémihydrate, isomère (-).

On prépare ce composé selon le mode opératoire décrit à l'étape B de l'Exemple 4 à partir de 0,58 g du composé obtenu à l'étape précédente dans 50 ml de DCM, 0,23 g du composé obtenu à la Préparation 3.1 (base libre), 0,58 g de triacétoxyborohydrure de sodium et 8 gouttes d'acide acétique. On obtient 0,4 g du produit attendu.

$\alpha_D^{20}$ = -23,4° (c = 0,5 ; MeOH).

RMN $^1H$ : δ (ppm) : 0,6 à 1,8 : m : 18H ; 1,8 à 4,8 : m : 19H ; 6,4 à 8,0 : m : 8H ; 9,8 à 10,2 : se : 1H.

EXEMPLE 6

Chlorhydrate de 2-[2-[4-(1-carbamoylcyclohexyl)pipéridin-1-yl]éthyl]-2-(3,4-dichlorophényl)-4-[2-[3,5-bis(trifluorométhyl)phényl]acétyl]morpholine, isomère (+).

(I), HCl : X = 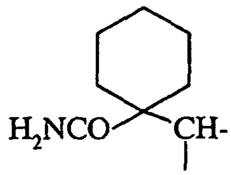 ; $R_1 = CF_3$ ; Ar = 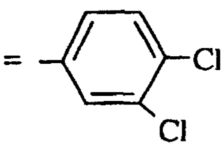

A) 2-(3,4-Dichlorophényl)-2-(formylméthyl)-4-[2-[3,5-bis(trifluorométhyl)phényl] acétyl)morpholine, isomère unique.

On prépare ce composé selon le mode opératoire décrit à l'étape A de l'Exemple 4 à partir de 0,62 ml de chlorure d'oxalyle dans 15 ml de DCM, 1,26 ml de DMSO dans 15 ml de DCM, 3,15 g du composé obtenu à l'étape B de l'Exemple 3 et 1,81 ml de DMSO dans 15 ml de DCM et 5,12 ml de triéthylamine. On obtient 3,13 g du produit attendu.

B) Chlorhydrate de 2-[2-[4-(1-carbamoylcyclohexyl)pipéridin-1-yl]éthyl]-2-(3,4-dichlorophényl)-4-[2-[3,5-bis(trifluorométhyl)phényl]acétyl]morpholine, isomère (+).

On prépare ce composé selon le mode opératoire décrit à l'étape B de l'Exemple 4 à partir de 0,5 g du composé obtenu à l'étape précédente dans 20 ml de DCM, 0,198 g du composé obtenu à la Préparation 3.5 (base libre), 0,46 g de triacétoxyborohydrure de sodium et 7 gouttes d'acide acétique. On obtient 0,467 g du produit attendu.
$\alpha_D^{20}$ = +28,8° (c = 0,5 ; MeOH).

RMN $^1H$ : δ (ppm) : 0,6 à 1,9 : m : 12H ; 1,9 à 4,5 : m : 18H ; 6,8 à 8,2 : m : 8H ; 9,8 à 10,4 : 2s : 2H.

En procédant selon les modes opératoires décrites dans les Exemples précédents, on prépare les composés selon l'invention rassemblés dans le Tableau I ci-après.

TABLEAU I

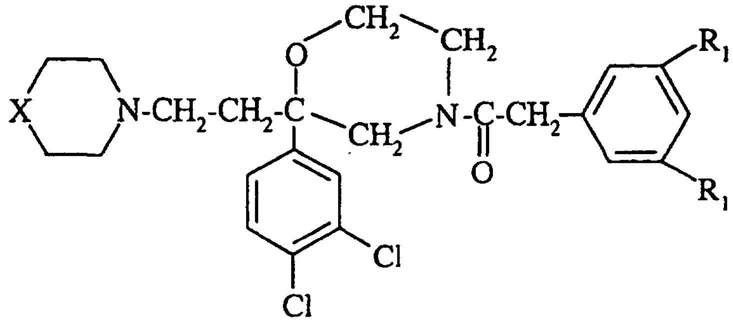

(I)

| Exemples | X | $R_1$ | Sel, hydrate<br>RMN<br>$\alpha_D^{20}$ (c= 0,5 ; MeOH) |
|---|---|---|---|
| 7<br>(a) | 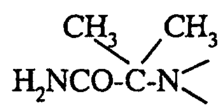 | $CH_3$ | 2HCl, 1,5 $H_2O$<br>RMN<br>-27° |
| 8<br>(b) | 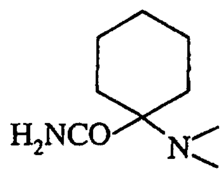 | $-CH_3$ | 2HCl, 1 $H_2O$<br>RMN<br>-24,6° |
| 9<br>(c) | 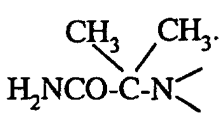 | $-CH_2CH_3$ | 2HCl, 0,75 $H_2O$<br>RMN<br>-24,2° |
| 10<br>(d) | 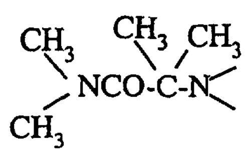 | $-CH_2CH_3$ | 2HCl, 0,5 $H_2O$<br>RMN<br>-23,6° |
| 11<br>(e) | 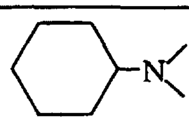 | $-CH_2CH_3$ | 2HCl, 0,5 $H_2O$<br>RMN<br>-25,2° (c=0,25) |

| Exemples | X | $R_1$ | Sel, hydrate RMN $\alpha_D^{20}$ (c= 0,5 ; MeOH) |
|---|---|---|---|
| 12 (f) | 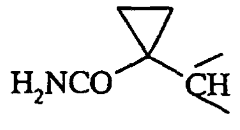 | $-CH_3$ | HCl, 2 $H_2O$ RMN -24,4° |
| 13 (g) | 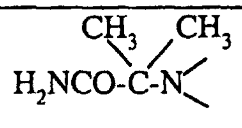 | $CF_3$ | 2HCl, 2 $H_2O$ RMN +27,6° |

(a) On prépare ce composé selon le mode opératoire décrit à l'étape B de l'Exemple 4 à partir du composé obtenu à l'étape A de l'Exemple 4 et du composé obtenu à la Préparation 3.2 sous forme de base libre.

(b) On prépare ce composé selon le mode opératoire décrit à l'étape B de l'Exemple 4 à partir du composé obtenu à l'étape A de l'Exemple 4 et du composé obtenu à la Préparation 3.3 sous forme de base libre.

(c) On prépare ce composé selon le mode opératoire décrit à l'étape D de l'Exemple 5 à partir du composé obtenu à l'étape C de l'Exemple 5 et du composé obtenu à la Préparation 3.2 sous forme de base libre.

(d) On prépare ce composé selon le mode opératoire décrit à l'étape D de l'Exemple 5 à partir du composé obtenu à l'étape C de l'Exemple 5 et du composé obtenu à la Préparation 3.4 sous forme de base libre.

(e) On prépare ce composé selon le mode opératoire décrit à l'étape D de l'Exemple 5 à partir du composé obtenu à l'étape C de l'Exemple 5 et de 1-cyclohexylpipérazine.

(f) On prépare ce composé selon le mode opératoire décrit à l'étape B de l'Exemple 4 à partir du composé obtenu à l'étape A de l'Exemple 4 et du composé obtenu à la Préparation 3.6 sous forme de base libre.

(g) On prépare ce composé selon le mode opératoire décrit à l'étape B de l'Exemple 4 à partir du composé obtenu à l'étape A de l'Exemple 6 et du composé obtenu à la Préparation 3.2 sous forme de base libre.

EXEMPLE 7 : RMN $^1H$ : δ (ppm) : 1,6 : se : 6H ; 2,0 à 2,4 : m : 8H ; 2,5 à 5,0 : m : 18H ; 6,6 à 8,0 : m : 10H.

EXEMPLE 8 : RMN $^1H$ : δ (ppm) : 0,8 à 2,4 : m : 18H ; 2,6 à 4,8 : m : 18H ; 6,4 à 8,2 : m : 8H.

EXEMPLE 9 : RMN $^1H$ : δ (ppm) : 0,8 à 1,2 : 2t : 6H ; 1,4 : se : 6H ; 2,0 à 5,0 : m : 24H ; 6,4 à 8,0 : m : 6H.

EXEMPLE 10 : RMN $^1H$ : δ (ppm) : 0,6 à 1,8 : m : 12H ; 2,0 à 4,8 : m : 32H ; 6,4 à 8,0 : m : 6H ; 10,6 à 11 : se : 2H.

EXEMPLE 11 : RMN $^1H$ : δ (ppm) : 0,9 à 2,7 : m : 22H ; 2,7 à 4,8 : m : 19H ; 6,4 à 7,8 : m : 6H ; 11,75 : s : 1H.

EXEMPLE 12 : RMN $^1H$ : δ (ppm) : 0,4 à 1,0 : 2mt : 4H ; 1,3 à 2,5 : m : 13H ; 2,55 à 4,5 : m : 14H ; 6,4 à 7,8 : m : 8H ; 10,1 : s : 1H.

EXEMPLE 13 : RMN $^1H$ : δ (ppm) : 1,4 : se : 6H ; 2,15 à 4,4 : m : 20H ; 7,2 à 8,2 : m : 8H.

EXEMPLE 14

Chlorhydrate de 2-[2-[4-(1-carbamoyl-1-méthyléthyl)pipéridin-1-yl]éthyl]-2-(3,4-dichlorophényl)-4-[2-(3,5-dichlorophényl)acétyl]morpholine, hémihydrate, isomère (+).

(I), HCl : X = $H_2NCO-C(CH_3)_2-CH-$ ; $R_1$ = Cl ; Ar = 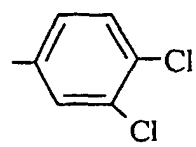

A) 2-(3,4-Dichlorophényl-2-(formylméthyl)-4-[2-(3,5-dichlorophényl)acétyl] morpholine, isomère unique.

On prépare ce composé selon le mode opératoire décrit à l'étape A de l'Exemple 4 à partir de 0,715 g de chlorure d'oxalyle dans 15 ml de DCM, 1,08 g de DMSO dans 15 ml de DCM, 2,14 g du composé obtenu à l'étape B de l'Exemple 2 et 1,55 g de DMSO dans 15 ml de DCM et 2,89 g de triéthylamine. On obtient 2,13 g du produit attendu.

B) Chlorhydrate de 2-[2-[4-(1-carbamoyl-1-méthyléthyl)pipéridin-1-yl]éthyl]-2-(3,4-dichlorophényl)-4-[2-(3,5-dichlorophényl)acétyl]morpholine, hémihydrate, isomère (+).

On prépare ce composé selon le mode opératoire décrit à l'étape B de l'Exemple 4 à partir de 0,47 g du composé obtenu à l'étape précédente dans 20 ml de DCM, 0,21 g du composé obtenu à la Préparation 3.1 (base libre), 0,5 g de triacétoxyborohydrure de sodium et 8 gouttes d'acide acétique. On obtient 0,428 g du produit attendu.
$\alpha_D^{20}$ = +4,8° (c = 0,5 ; MeOH)

RMN $^1H$ : δ (ppm) : 0,9 : s : 6H ; 1,3 à 2,5 : m : 7H ; 2,5 à 4,2 : m : 14H ; 6,6 à 7,8 : m : 8H ; 10,1 :2s : 1H.

EXEMPLE 15

Chlorhydrate de 2-[2-[4-(1-carbamoyl-1-méthyléthyl)pipéridin-1-yl]éthyl]-2-(3,4-dichlorophényl)-4-[2-[3,5-bis(trifluorométhyl)phényl]acétyl]morpholine, monohydrate, isomère (+).

(I), HCl : X = $H_2NCO-C(CH_3)_2-CH-$ ; $R_1 = CF_3$ ; Ar = 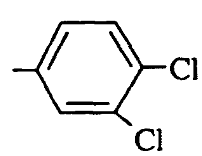

On chauffe à reflux pendant 3 heures un mélange de 0,3 g du composé obtenu à la Préparation 3.1 et 0,6 g

de $K_2CO_3$ dans 10 ml d'acétonitrile. On filtre un insoluble et concentre sous vide le filtrat. On dissout le produit de la Préparation 3.1 sous forme de base libre ainsi obtenu dans 20 ml de DCM, ajoute 0,77 g du composé obtenu à l'étape A de l'Exemple 6, puis 0,62 g de triacétoxyborohydrure de sodium et 8 gouttes d'acide acétique et laisse une nuit sous agitation à TA et sous atmosphère d'azote. On alcalinise le mélange réactionnel à pH = 8 par ajout d'une solution de $Na_2CO_3$ à 10 %, extrait au DCM, lave trois fois la phase organique à l'eau, par une solution saturée de NaCl, sèche sur $MgSO_4$ et évapore sous vide le solvant. On chromatographie le résidu sur gel de silice H en éluant au DCM, puis par le mélange DCM/MeOH (95/5 ; v/v). On dissout le produit obtenu dans de l'AcOEt, ajoute de l'éther chlorhydrique jusqu'à pH = 1, essore le précipité formé et le sèche. On obtient 0,5 g du produit attendu.
$\alpha_D^{20}$ = +29° (c = 0,5 ; MeOH)

RMN $^1H$ : $\delta$ (ppm) : 1,0 : s : 6H ; 1,4 à 2,5 : m : 6H ; 2,5 à 4,4 : m : 15H ; 6,8 à 7,2 : 2s : 2H ; 7,3 à 8,1 : m : 6H ; 9,7 à 10,15 :2s : 1H.

EXEMPLE 16 Dichlorhydrate de 2-[2-[4-(1-carbamoyl-1-méthyléthyl)pipérazin-1-yl]éthyl]-2-(3,4-dichlorophényl)-4-[2-(3,5-diisopropylphényl)acétyl]morpholine, monohydrate, isomère (-).

(I), 2HCl : X = 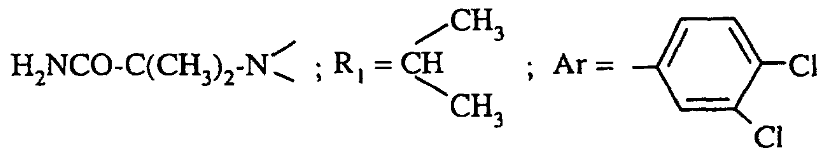

A) 2-(3,4-Dichlorophényl-2-(2-hydroxyéthyl)-4-[2-(3,5-diisopropylphényl)acétyl] morpholine, isomère unique.

On refroidit à 0°C un mélange de 1,78 g du composé obtenu à la Préparation 1.2, 0,8 g de triéthylamine et 1,4 g d'acide 3,5-diisopropylphénylacétique dans 40 ml de DCM, ajoute 2,85 g de BOP et laisse 30 minutes sous agitation à TA On concentre sous vide le mélange réactionnel, extrait le résidu à l'éther, lave la phase organique à l'eau, par une solution tampon pH = 2, à l'eau, par une solution de NaOH 1N, à l'eau, sèche sur $Na_2SO_4$ et évapore sous vide le solvant. On chromatographie le résidu sur gel de silice H en éluant par le gradient du mélange DCM/MeOH de (100/1 ; v/v) à (100/3 ; v/v). On obtient 1,2 g du produit attendu.

B) 2-(3,4-Dichlorophényl-2-(formylméthyl)-4-[2-(3,5-diisopropylphényl)acétyl] morpholine, isomère unique

On prépare ce composé selon le mode opératoire décrit à l'étape A de l'Exemple 4 à partir de 0,4 g de chlorure d'oxalyle dans 20 ml de DCM, 0,6 g de DMSO dans 10 ml de DCM, 1,2 g du composé obtenu à l'étape précédente et 0,8 g de DMSO dans 20 ml de DCM et 1,64 g de triéthylamine. On obtient 1,1 g du produit attendu.

C)Dichlorhydratede2-[2-[4-(1-carbamoyl-1-méthyléthyl)pipérazin-1-yl]éthyl]-2-(3,4-dichlorophényl)-4-[2-(3,5-diisopropylphényl)acétyl]morpholine, monohydrate, isomère (-).

On prépare ce composé selon le mode opératoire décrit à l'étape B de l'Exemple 4 à partir de 0,45 g du composé obtenu à l'étape précédente dans 50 ml de DCM, 0,2 g du composé obtenu à la Préparation 3.2 sous forme de base libre, 0,4 g de triacétoxyborohydrure de sodium et 8 gouttes d'acide acétique. On obtient 0,3 g du produit attendu.
$\alpha_D^{20}$ = -18,4° (c = 0,25 ; MeOH)

RMN $^1H$ : $\delta$ (ppm) : 0,8 à 1,7 : m : 18H ; 2,2 : mt : 2H ; 2,5 à 4,7 : m : 18H ; 6,4 8,0 : m : 8H.

## Revendications

1. Composé de formule :

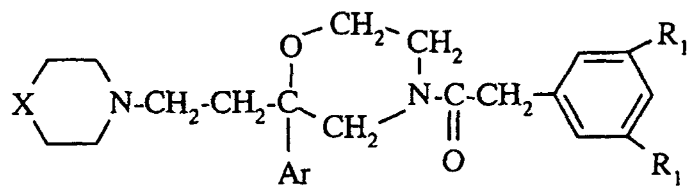

(I)

dans laquelle :

- Ar représente un phényle monosubstitué ou disubstitué par un atome d'halogène ; un $(C_1\text{-}C_3)$alkyle ;
- X représente un groupe

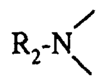 ;

un groupe

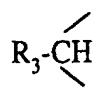 ;

- $R_1$ représente un atome de chlore, un atome de brome, un $(C_1\text{-}C_3)$alkyle ou un trifluorométhyle ;
- $R_2$ représente un $(C_1\text{-}C_6)$alkyle ; un $(C_3\text{-}C_6)$cycloalkyle ; un groupe
- $CR_4R_5CONR_6R_7$ ;
- $R_3$ représente un groupe $-CR_4R_5CONR_6R_7$ ;
- $R_4$ et $R_5$ représentent le même radical choisi parmi un méthyle, un éthyle, un n-propyle ou un n-butyle ;
- ou bien $R_4$ et $R_5$ ensemble avec l'atome de carbone auquel ils sont liés constituent un $(C_3\text{-}C_6)$cycloalkyle ;
- $R_6$ et $R_7$ représentent chacun indépendamment un hydrogène ; un $(C_1\text{-}C_3)$alkyle;
- ou bien $R_6$ et $R_7$ ensemble avec l'atome d'azote auquel ils sont liés constituent un radical hétérocyclique choisi parmi l'azétidin-1-yle, la pyrrolidin-1-yle, la pipéridin-1-yle, la morpholin-4-yle, la thiomorpholin-4-yle ou la perhydroazépin-1-yle ;

et ses sels avec des acides minéraux ou organiques, ses solvats et/ou ses hydrates.

**2.** Composé selon la revendication 1 dans lequel Ar représente un 3,4-dichlorophényle.

**3.** Composé selon la revendication 1 dans lequel les substituants $R_1$ représentent un atome de chlore, un méthyle, un éthyle, un isopropyle ou un trifluorométhyle.

**4.** Composé selon la revendication 1 dans lequel X représente un groupe

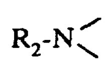

dans lequel $R_2$ représente un $(C_1\text{-}C_6)$ alkyle ou un $(C_3\text{-}C_6)$cycloalkyle.

**5.** Composé selon la revendication 4 dans lequel $R_2$ représente un cyclopentyle ou un cyclohexyle.

**6.** Composé selon la revendication 1 dans lequel X représente un groupe

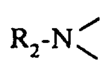

dans lequel $R_2$ représente un groupe $-CR_4R_5CONR_6R_7$.

**7.** Composé selon la revendication 6 dans lequel $R_4$ et $R_5$ représentent chacun un méthyle ou bien, ensemble avec l'atome de carbone auquel ils sont liés, constituent un cyclohexyle.

**8.** Composé selon la revendication 6 dans lequel $R_6$ et $R_7$ sont semblables et représentent l'hydrogène ou un méthyle.

**9.** Composé selon la revendication 1 dans lequel X représente un groupe

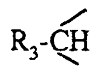

dans lequel $R_3$ représente un groupe $-CR_4R_5CONR_6R_7$.

**10.** Composé selon la revendication 9 dans lequel $R_4$ et $R_5$ représentent chacun un méthyle ou bien, ensemble avec l'atome de carbone auquel ils sont liés, constituent un cyclohexyle ou un cyclopropyle.

**11.** Composé selon la revendication 9 dans lequel $R_6$ et $R_7$ sont semblables et représentent l'hydrogène ou un méthyle.

**12.** Composé selon la revendication 1 de formule :

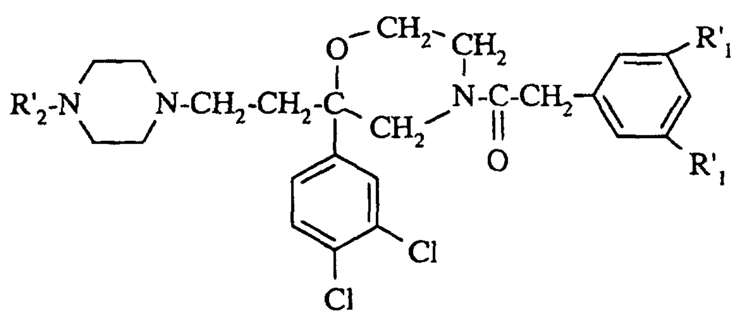

(I')

dans laquelle :

- $R'_1$ représente un atome de chlore, un méthyle, un éthyle, un isopropyle ou un trifluorométhyle ;
- $R'_2$ représente un cyclopentyle ou un cyclohexyle ;

et ses sels avec des acides minéraux ou organiques, ses solvats et/ou ses hydrates.

**13.** Composé selon la revendication 1 de formule :

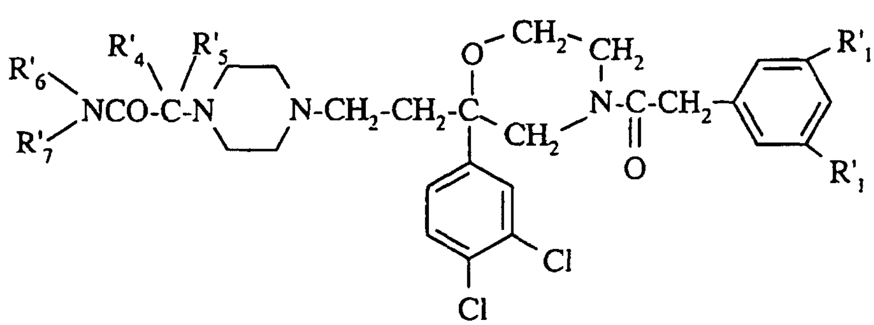

(I")

dans laquelle :

- $R'_1$ représente un atome de chlore, un méthyle, un éthyle, un isopropyle ou un trifluorométhyle ;
- $R'_4$ et $R'_5$ représentent chacun un méthyle ou bien, ensemble avec l'atome de carbone auquel ils sont liés

constituent un cyclohexyle ;

- $R'_6$ et $R'_7$ sont semblables et représentent l'hydrogène ou un méthyle ;

et ses sels avec des acides minéraux ou organiques, ses solvats et/ou ses hydrates.

**14.** Composé selon la revendication 1 de formule :

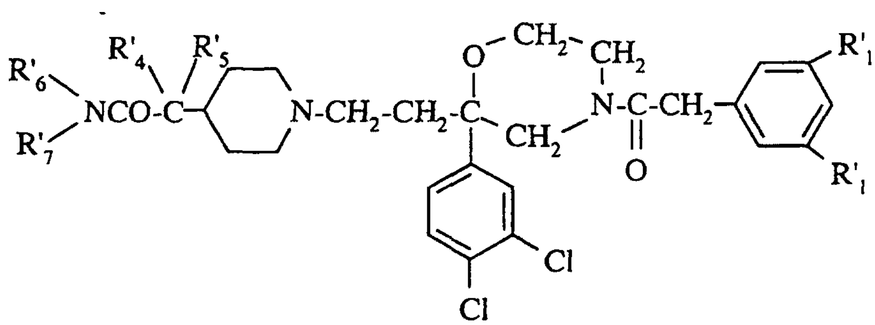

(I''')

dans laquelle :

- $R'_1$ représente un atome de chlore, un méthyle, un éthyle, un isopropyle ou un trifluorométhyle ;
- $R'_4$ et $R'_5$ représentent chacun un méthyle ou bien, ensemble avec l'atome de carbone auquel ils sont liés, constituent un cyclohexyle ou un cyclopropyle ;
- $R'_6$ et $R'_7$ sont semblables et représentent l'hydrogène ou un méthyle ;

et ses sels avec des acides minéraux ou organiques, ses solvats et/ou ses hydrates.

**15.** Composé selon l'une quelconque des revendications 1 à 14 de formule (I), (I'), (I'') ou (I''') sous forme optiquement pure.

**16.** La 2-[2-(4-cyclohexylpipérazin-1-yl)éthyl]-2-(3,4-dichlorophényl)-4-[2-[3,5-bis(trifluorométhyl)phényl]acétyl]morpholine, isomère (+), ses sels, ses solvats et/ou ses hydrates.

**17.** La 2-[2-[4-(1-carbamoylcyclohexyl)pipéridin-1-yl]éthyl]-2-(3,4-dichlorophényl)-4-[2-[3,5-bis(trifluorométhyl)phényl]acétyl]morpholine, isomère (+), ses sels, ses solvats et/ou ses hydrates.

**18.** La 2-[2-[4-(1-carbamoyl-1-méthyléthyl)pipérazin-1-yl]éthyl]-2-(3,4-dichloro phényl)-4-[2-[3,5-bis(trifluorométhyl) phényl]acétyl]morpholine, isomère (+), ses sels, ses solvats et/ou ses hydrates.

**19.** La 2-[2-[4-(1-carbamoyl-1-méthyléthyl)pipéridin-1-yl]éthyl]-2-(3,4-dichloro phényl)-4-[2-[3,5-bis(trifluorométhyl) phényl]acétyl]morpholine, isomère (+), ses sels, ses solvats et/ou ses hydrates.

**20.** Procédé pour la préparation des composés de formule (I) selon la revendication 1, de leurs sels, leurs solvats et/ou leurs hydrates, **caractérisé en ce que** :

1a) on traite un composé de formule :

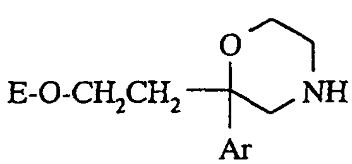

(II)

dans laquelle Ar est tel que défini pour un composé de formule (I) dans la revendication 1 et E représente l'hydrogène ou un groupe O-protecteur, avec un dérivé fonctionnel d'un acide de formule :

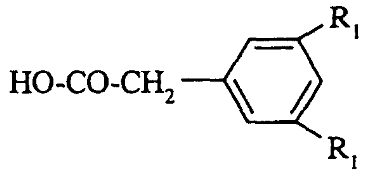

(III)

dans laquelle $R_1$ est tel que défini pour un composé de formule (I) dans la revendication 1, pour obtenir un composé de formule :

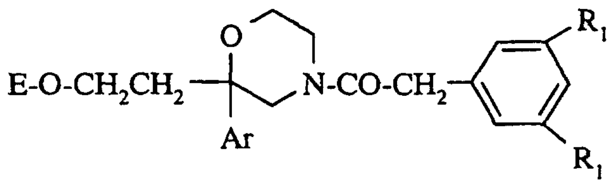

(IV) ;

2a) éventuellement, lorsque E représente un groupe protecteur, on l'élimine par action d'un acide ou d'une base, pour obtenir l'alcool de formule :

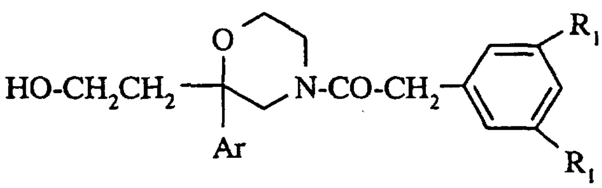

(IV, E = H) ;

3a) on traite l'alcool obtenu à l'étape 1a) ou à l'étape 2a) de formule (IV, E = H) avec un composé de formule :

$$Y\text{-}SO_2\text{-}Cl \quad (V)$$

dans laquelle Y représente un groupe méthyle, phényle, tolyle, trifluorométhyle, pour obtenir un composé de formule :

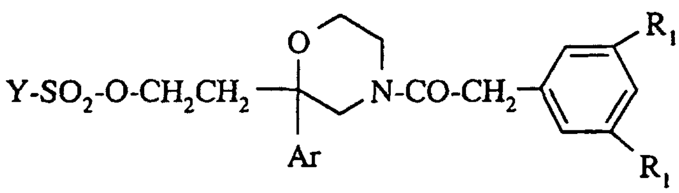

(VI)

4a) on fait réagir le composé de formule (VI) avec un composé de formule :

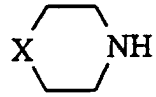

(VII)

dans laquelle X est tel que défini pour un composé de formule (I) dans la revendication 1;
5a) et, éventuellement, on transforme le composé ainsi obtenu en l'un de ses sels avec un acide minéral ou organique.

**21.** Procédé pour la préparation des composés de formule (I) selon la revendication 1, de leurs sels, leurs solvats et/ou leurs hydrates, **caractérisé en ce que** :

1b) on traite le composé de formule :

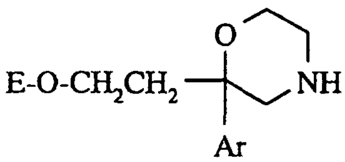

(II)

dans laquelle Ar est tel que défini pour un composé de formule (I) dans la revendication 1, E représente l'hydrogène ou un groupe O-protecteur, avec un dérivé fonctionnel d'un acide de formule :

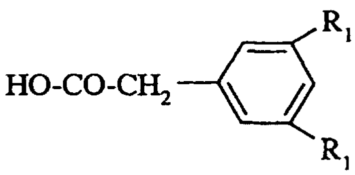

(III)

dans laquelle $R_1$ est tel que défini pour un composé de formule (I) dans la revendication 1, pour obtenir un composé de formule :

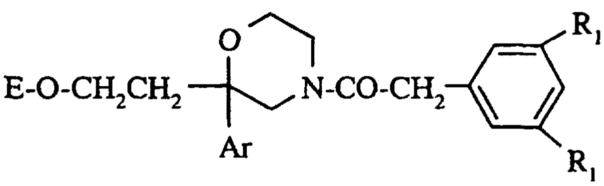

(IV) ;

éventuellement, lorsque E représente un groupe protecteur, on l'élimine par action d'un acide ou d'une base, pour obtenir l'alcool de formule :

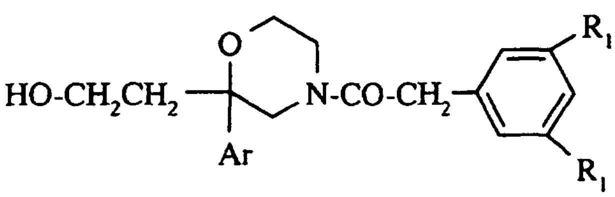

(IV, E = H) ;

2b) on oxyde le composé de formule (IV, E = H) ainsi obtenu pour préparer un composé de formule :

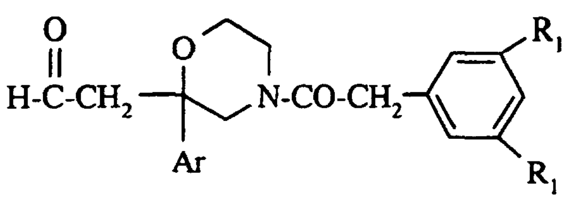

(VIII)

3b) on fait réagir le composé de formule (VIII) avec un composé de formule :

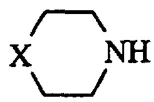 (VII)

dans laquelle X est tel que défini pour un composé de formule (I) dans la revendication 1, en présence d'un acide, puis on réduit le sel d'imminium formé intermédiairement au moyen d'un agent réducteur ;

4b) et, éventuellement, on transforme le composé ainsi obtenu en un de ses sels avec un acide minéral ou organique.

**22.** Composition pharmaceutique comprenant en tant que principe actif un composé selon l'une quelconque des revendications 1 à 19 ou l'un de ses sels, solvats et/ou hydrates pharmaceutiquement acceptables.

**23.** Composition pharmaceutique selon le revendication 22, contenant de 0,1 à 1000 mg de principe actif, sous forme d'unité de dosage dans laquelle le principe actif est mélangé à au moins un excipient pharmaceutique.

**24.** Utilisation des composés de formule (I) selon la revendication 1 ou l'un de leurs sels, solvats et/ou hydrates pharmaceutiquement acceptables pour la préparation de médicaments destinés à traiter toute pathologie où la Substance P et/ou les récepteurs $NK_1$ humains sont impliqués.

**25.** Utilisation selon la revendication 24, pour la préparation de médicaments destinés à traiter les pathologies du système respiratoire, gastro-intestinal, urinaire, immunitaire, cardiovasculaire, du système nerveux central ainsi que la douleur, la migraine, les inflammations, les nausées et les vomissements, les maladies de la peau.

**26.** Utilisation selon la revendication 25 pour la préparation de médicaments destinés à traiter la bronchite chronique obstructive, l'asthme, l'incontinence urinaire, le syndrome du colon irritable, la maladie de Crohn, les colites ulcératives, la dépression, l'anxiété, l'épilepsie.

**27.** Médicament **caractérisé en ce qu'**il est constitué d'un composé selon l'une quelconque des revendications 1 à 19.

**Patentansprüche**

**1.** Verbindung der Formel:

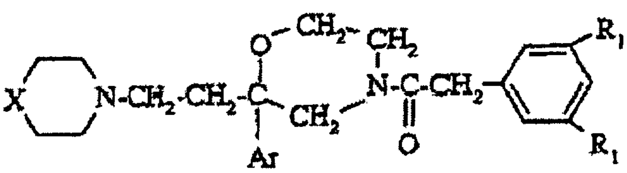 (I)

in der:

- Ar durch ein Halogenatom; oder ($C_1$-$C_3$)-Alkyl monosubstituiertes oder disubstituiertes Phenyl bedeutet;
- X eine Gruppe

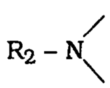 ;

oder eine Gruppe

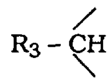

darstellt;

- $R_1$ ein Chloratom, ein Bromatom, $(C_1-C_3)$-Alkyl- oder Trifluormethyl bedeutet;
- $R_2$ $(C_1-C_6)$-Alkyl; $(C_3-C_6)$-Cycloalkyl; oder $-CR_4R_5CONR_6R_7$ bedeutet;
- $R_3$ eine Gruppe $-CR_4R_5CONR_6R_7$ darstellt;
- $R_4$ und $R_5$ die gleiche Gruppe ausgewählt aus Methyl, Ethyl, n-Propyl oder n-Butyl bedeuten;
- oder $R_4$ und $R_5$ gemeinsam mit dem Kohlenstoffatom, an das sie gebunden sind, $(C_3-C_6)$-Cycloalkyl bilden;
- $R_6$ und $R_7$ jeweils unabhängig voneinander Wasserstoff; oder $(C_1-C_3)$-Alkyl bedeuten;
- oder $R_6$ und $R_7$ zusammen mit dem Stickstoffatom, an das sie gebunden sind, einen heterocyclischen Rest bilden, ausgewählt aus Azetidin-1-yl. Pyrrolidin-1-yl, Piperidin-1-yl, Morpholin-4-yl, Thiomorpholin-4-yl oder Perhydroazepin-1-yl;

und deren Salze mit anorganischen oder organischen Säuren, deren Solvate und/oder deren Hydrate.

**2.** Verbindung nach Anspruch 1, worin Ar 3,4-Dichlorphenyl bedeutet.

**3.** Verbindung nach Anspruch 1, worin die Substituenten $R_1$ ein Chloratom, Methyl, Ethyl, Isopropyl oder Trifluormethyl bedeuten.

**4.** Verbindung nach Anspruch 1, worin X eine Gruppe

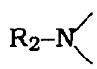

darstellt, in der $R_2$ $(C_1-C_6)$-Alkyl oder $(C_3-C_6)$-Cycloalkyl bedeutet.

**5.** Verbindung nach Anspruch 4, worin $R_2$ Cyclopentyl oder Cyclohexyl darstellt.

**6.** Verbindung nach Anspruch 1, worin X eine Gruppe

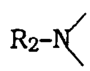

bedeutet, in der $R_2$ eine Gruppe $-CR_4R_5CONR_6R_7$ darstellt.

**7.** Verbindung nach Anspruch 6, worin $R_4$ und $R_5$ jeweils Methyl bedeuten oder gemeinsam mit dem Kohlenstoffatom, an das sie gebunden sind, Cyclohexyl bilden.

**8.** Verbindung nach Anspruch 6, worin $R_6$ und $R_7$ ähnlich sind und Wasserstoff oder Methyl bedeuten.

**9.** Verbindung nach Anspruch 1, worin X eine Gruppe

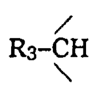

darstellt, in der $R_3$ eine Gruppe $-CR_4R_5CONR_6R_7$ bedeutet.

**10.** Verbindung nach Anspruch 9, worin $R_4$ und $R_5$ jeweils Methyl bedeuten oder zusammen mit dem Kohlenstoffatom, an das sie gebunden sind, Cyclohexyl oder Cyclopropyl bilden.

**11.** Verbindung nach Anspruch 9, worin $R_6$ und $R_7$ ähnlich sind und Wasserstoff oder Methyl bedeuten.

**12.** Verbindung nach Anspruch 1 der Formel:

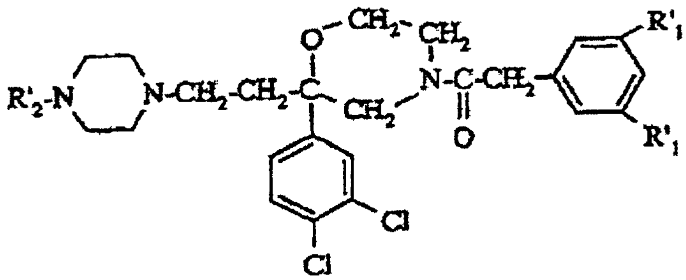

(I')

in der:

- R'$_1$ ein Chloratom, Methyl, Ethyl, Isopropyl oder Trifluormethyl;
- R'$_2$ Cyclopentyl oder Cyclohexyl bedeuten;

deren Salze mit anorganischen oder organischen Säuren, deren Solvate und/oder deren Hydrate.

**13.** Verbindung nach Anspruch 1 der Formel:

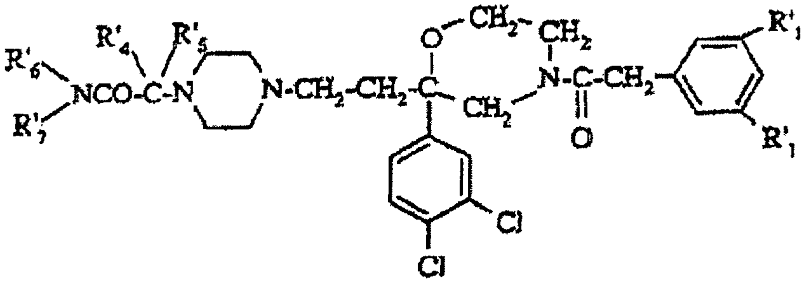

(I'')

in der:

- R'$_1$ ein Chloratom, Methyl, Ethyl, Isopropyl oder Trifluormethyl bedeutet;
- R'$_4$ und R'$_5$ jeweils Methyl bedeuten oder gemeinsam mit dem Kohlenstoffatom, an das sie gebunden sind, Cyclohexyl bilden;
- R'$_6$ und R'$_7$ ähnlich sind und Wasserstoff oder Methyl bedeuten;

und deren Salze mit anorganischen oder organischen Säuren, deren Solvate und/oder deren Hydrate.

**14.** Verbindung nach Anspruch 1 der Formel:

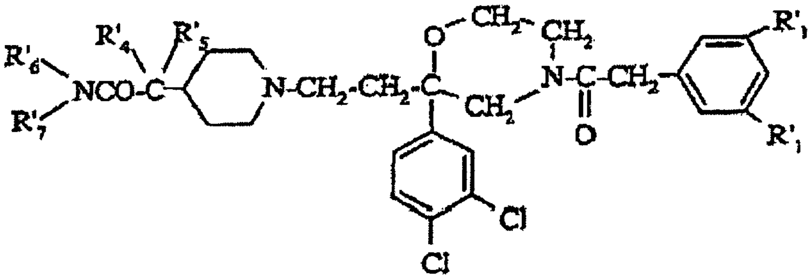

(I''')

in der:

- $R'_1$ ein Chloratom, Methyl, Ethyl. Isopropyl oder Trifluormethyl darstellt;
- $R'_4$ und $R'_5$ jeweils Methyl bedeuten oder gemeinsam mit dem Kohlenstoffatom, an das sie gebunden sind, Cyclohexyl oder Cyclopropyl bilden;
- $R'_6$ und $R'_7$ ähnlich sind und Wasserstoff oder Methyl bedeuten;

und deren Salze mit anorganischen oder organischen Säuren, deren Solvate und/oder deren Hydrate.

**15.** Verbindung nach einem der Ansprüche 1 bis 14 der Formel (I), (I'), (I'') oder (I''') in optisch reiner Form.

**16.** 2-[2-(4-Cyclohexylpiperazin-1-yl)-ethyl]-2-(3,4-dichlorphenyl)-4-[2-[3,5-bis(trifluormethyl)-phenyl]-acetyl]-morpholin, Isomeres (+), dessen Salze, dessen Solvate und/oder dessen Hydrate.

**17.** 2-[2-[4-(1-Carbamoylcyclohexyl)-piperidin-1-yl]-ethyl]-2-(3,4-dichlorphenyl)-4-[2-[3,5-bis(trifluormethyl)-phenyl]-acetyl]-morpholin, Isomeres (+), dessen Salze, dessen Solvate und/oder dessen Hydrate.

**18.** 2-[2-[4-(1-Carbamoyl-1-methylethyl)-piperazin-1-yl]-ethyl]-2-(3,4-dichlorphenyl)-4-[2-[3,5-bis(trifluormethyl)-phenyl]-acetyl]-morpholin, Isomeres (+), dessen Salze, dessen Solvate und/oder dessen Hydrate.

**19.** 2-[2-[4-(1-Carbamoyl-1-methylethyl)-piperidin-1-yl]-ethyl]-2-(3,4-dichlorphenyl)-4-[2-[3,5-bis(trifluormethyl)-phenyl]-acetyl]-morpholin, Isomeres (+), dessen Salze, dessen Solvate und/oder dessen Hydrate.

**20.** Verfahren zur Herstellung der Verbindungen der Formel (I) nach Anspruch 1, von deren Salzen, von deren Solvaten und/oder von deren Hydraten, **dadurch gekennzeichnet, daß** man:

1a) eine Verbindung der Formel:

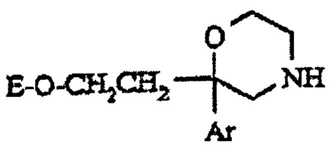

(II)

in der Ar die für eine Verbindung der Formel (I) in Anspruch 1 angegebenen Bedeutungen besitzt und E Wasserstoff oder eine O-Schutzgruppe bedeutet, mit einem funktionellen Derivat einer Säure der Formel:

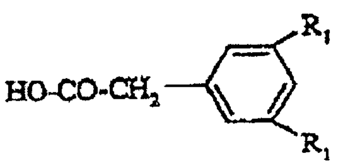

(III)

in der $R_1$ die für eine Verbindung der Formel (I) in Anspruch 1 angegebenen Bedeutungen besitzt, umsetzt zur Bildung einer Verbindung der Formel:

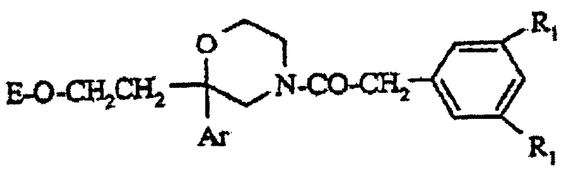

(IV) ;

2a) gegebenenfalls, wenn E eine Schutzgruppe bedeutet, diese durch Einwirkung einer Säure oder einer Base entfernt zur Bildung des Alkohols der Formel:

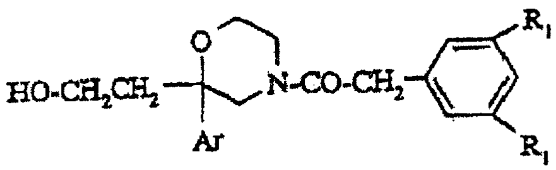 (IV, E = H) ;

3a) den in der Stufe 1a) oder in der Stufe 2a) erhaltenen Alkohol der Formel (IV, E = H) mit einer Verbindung der Formel:

$$Y\text{-}SO_2\text{-}Cl \quad (V)$$

in der Y eine Methyl-, Phenyl-, Tolyl- oder Trifluormethylgruppe bedeutet, behandelt zur Bildung einer Verbindung der Formel:

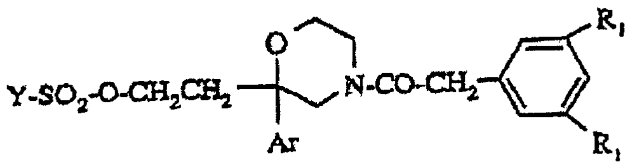 (VI)

4a) die Verbindung der Formel (VI) mit einer Verbindung der Formel:

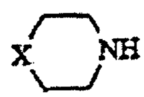 (VII)

in der X die für eine Verbindung der Formel (I) in Anspruch 1 angegebenen Bedeutungen besitzt, umsetzt; und
5a) gegebenenfalls die in dieser Weise erhaltene Verbindung in eines ihrer Salze mit einer anorganischen oder organischen Säure umwandelt.

**21.** Verfahren zur Herstellung der Verbindungen der Formel (I) nach Anspruch 1, von deren Salzen, deren Solvaten und/oder Hydraten, **dadurch gekennzeichnet, daß** man:

1b) die Verbindung der Formel:

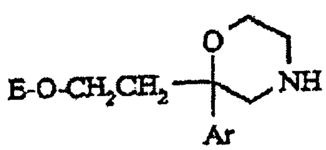 (II)

in der Ar die für eine Verbindung der Formel (I) in Anspruch 1 angegebenen Bedeutungen besitzt und E Wasserstoff oder eine O-Schutzgruppe darstellt, mit einem funktionellen Derivat einer Säure der Formel:

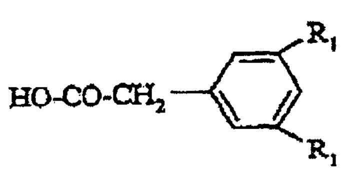 (III)

in der $R_1$ die für eine Verbindung der Formel (I) in Anspruch 1 angegebenen Bedeutungen besitzt, behandelt zur Bildung einer Verbindung der Formel:

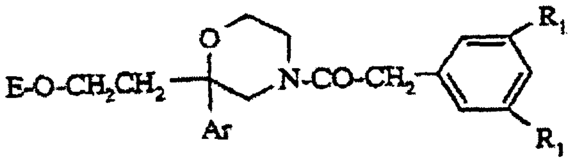

(IV) ;

gegebenenfalls, wenn E eine Schutzgruppe bedeutet, diese durch Einwirkung einer Säure oder einer Base entfernt zur Bildung des Alkohols der Formel:

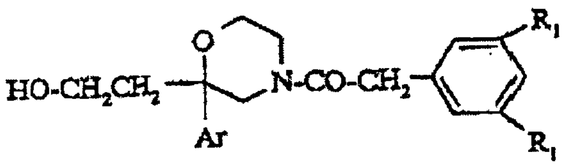

(IV, E = H) ;

2b) die in dieser Weise erhaltene Verbindung der Formel (IV, E = H) oxidiert zur Bildung einer Verbindung der Formel:

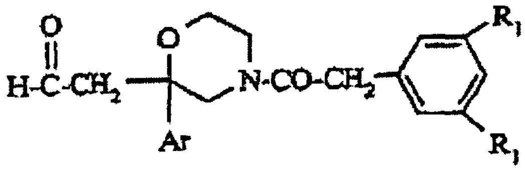

(VIII)

3b) die Verbindung der Formel (VIII) mit einer Verbindung der Formel:

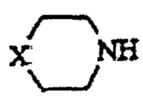

(VII)

in der X die für eine Verbindung der Formel (I) in Anspruch 1 angegebenen Bedeutungen besitzt, in Gegenwart einer Säure umsetzt und dann das als Zwischenprodukt gebildete Imminiumsalz mit Hilfe eines Reduktionsmittels reduziert; und

4b) gegebenenfalls die in dieser Weise erhaltene Verbindung in eines ihrer Salze mit einer anorganischen oder organischen Säure umwandelt.

**22.** Pharmazeutische Zubereitung, enthaltend als Wirkstoff eine Verbindung nach einem der Ansprüche 1 bis 19 oder eines ihrer pharmazeutisch annehmbaren Salze, Solvate und/oder Hydrate.

**23.** Pharmazeutische Zubereitung nach Anspuch 22, enthaltend 0,1 bis 1000 mg des Wirkstoffs in Einheitsdosisform, in der der Wirkstoff in Mischung mit mindestens einem pharmazeutischen Trägermaterials vorliegt.

**24.** Verwendung der Verbindungen der Formel (I) nach Anspruch 1 oder eines ihrer pharmazeutisch annehmbaren Salze, Solvate und/oder Hydrate für die Herstellung von Arzneimitteln zur Behandlung von sämtlichen pathologischen Zuständen, bei denen die Substanz P und/oder die menschlichen Rezeptoren $NK_1$ beteiligt sind.

**25.** Verwendung nach Anspruch 24 für die Herstellung von Arzneimitteln zur Behandlung von pathologischen Zuständen des Atmungssystems, des Magen-Darm-Systems, des Harnsystems, des Immunsystems, des kardiovaskulären Systems oder des Zentralnervensystems sowie von Schmerzen, Migräne, Entzündungszuständen, Übelkeit, Erbrechen und Hauterkrankungen.

**26.** Verwendung nach Anspruch 25 für die Herstellung von Arzneimitteln, die zur Behandlung der obstruktiven chronischen Bronchitis, des Asthmas, der Harninkontinenz, der Reizdarmsyndroms, der Crohnschen Krankheit, der Colitis ulcerosa, von Depression, der Angst und der Epilepsie.

**27.** Arzneimittel, **dadurch gekennzeichnet, daß** es aus einer Verbindung nach einem der Ansprüche 1 bis 19 gebildet ist.

## Claims

**1.** Compound of formula:

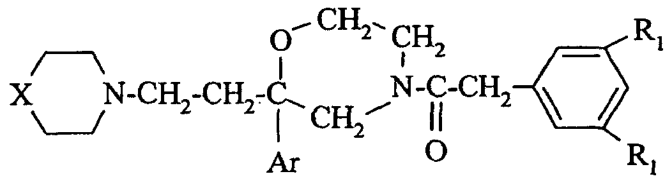

(I)

in which :

- Ar represents a phenyl monosubstituted or disubstituted with a halogen atom; a $(C_1-C_3)$alkyl;
- X represents a group

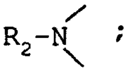

a group

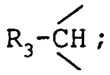

- $R_1$ represents a chlorine atom, a bromine atom, a $(C_1-C_3)$alkyl or a trifluoromethyl;
- $R_2$ represents a $(C_1-C_6)$alkyl; a $(C_3-C_6)$cycloalkyl; a group $-CR_4R_5CONR_6R_7$;
- $R_3$ represents a group $-CR_4R_5CONR_6R_7$;
- $R_4$ and $R_5$ represent the same radical chosen from a methyl, an ethyl, an n-propyl or an n-butyl;
- or alternatively $R_4$ and $R_5$, together with the carbon atom to which they are attached, constitute a $(C_3-C_6)$cycloalkyl;
- $R_6$ and $R_7$ each independently represent a hydrogen; a $(C_1-C_3)$alkyl;
- or alternatively $R_6$ and $R_7$, together with the nitrogen atom to which they are attached, constitute a heterocyclic radical chosen from 1-azetidinyl, 1-pyrrolidinyl, 1-piperidinyl, 4-morpholinyl, 4-thiomorpholinyl or perhydro-1-azepinyl; as well as the salts thereof with inorganic or organic acids, the solvates thereof and/or the hydrates thereof.

**2.** Compound according to Claim 1, in which Ar represents a 3,4-dichlorophenyl.

**3.** Compound according to Claim 1, in which the substituents $R_1$ represent a chlorine atom, a methyl, an ethyl, an isopropyl or a trifluoromethyl.

**4.** Compound according to Claim 1, in which X represents a group

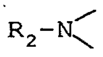

in which $R_2$ represents a $(C_1-C_6)$ alkyl or a $(C_3-C_6)$cycloalkyl.

**5.** Compound according to Claim 4, in which $R_2$ represents a cyclopentyl or a cyclohexyl.

**6.** Compound according to Claim 1, in which X represents a group

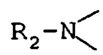

in which $R_2$ represents a group $-CR_4R_5CONR_6R_7$.

**7.** Compound according to Claim 6, in which $R_4$ and $R_5$ each represent a methyl or alternatively, together with the carbon atom to which they are attached, constitute a cyclohexyl.

**8.** Compound according to Claim 6, in which $R_6$ and $R_7$ are similar and represent hydrogen or a methyl.

**9.** Compound according to Claim 1, in which X represents a group

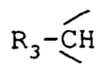

in which $R_3$ represents a group $-CR_4R_5CONR_6R_7$.

**10.** Compound according to Claim 9, in which $R_4$ and $R_5$ each represent a methyl or alternatively, together with the carbon atom to which they are attached, constitute a cyclohexyl or a cyclopropyl.

**11.** Compound according to Claim 9, in which $R_6$ and $R_7$ are similar and represent hydrogen or a methyl.

**12.** Compound according to Claim 1 of formula:

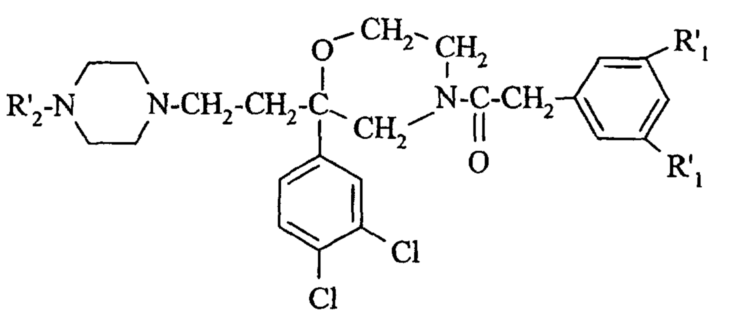

(I')

in which:

- $R'_1$ represents a chlorine atom, a methyl, an ethyl, an isopropyl or a trifluoromethyl;
- $R'_2$ represents a cyclopentyl or a cyclohexyl;

as well as the salts thereof with inorganic or organic acids, the solvates thereof and/or the hydrates thereof.

**13.** Compound according to Claim 1 of formula:

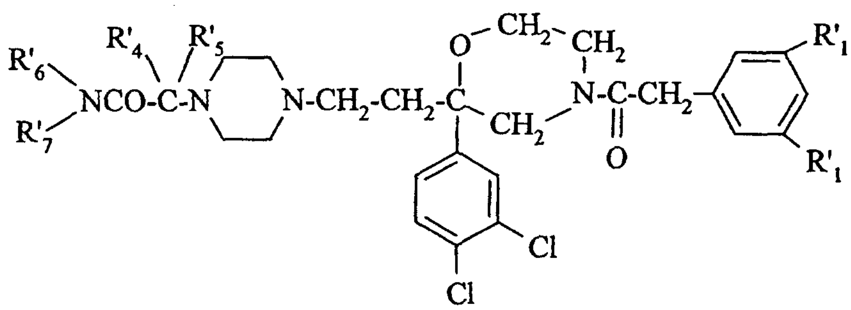

(I")

in which :

- $R'_1$ represents a chlorine atom, a methyl, an ethyl, an isopropyl or a trifluoromethyl;
- $R'_4$ and $R'_5$ each represent a methyl or alternatively, together with the carbon atom to which they are attached, constitute a cyclohexyl;
- $R'_6$ and $R'_7$ are similar and represent hydrogen or a methyl;

as well as the salts thereof with inorganic or organic acids, the solvates thereof and/or the hydrates thereof.

**14.** Compound according to Claim 1 of formula:

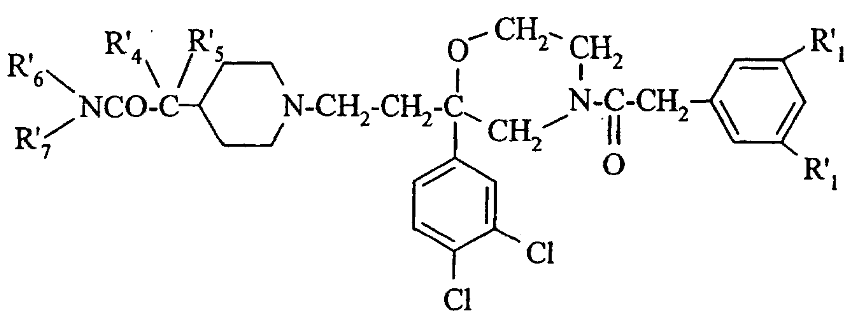

(I"')

in which:

- $R'_1$ represents a chlorine atom, a methyl, an ethyl, an isopropyl or a trifluoromethyl;
- $R'_4$ and $R'_5$ each represent a methyl or alternatively, together with the carbon atom to which they are attached, constitute a cyclohexyl or a cyclopropyl;
- $R'_6$ and $R'_7$ are similar and represent hydrogen or a methyl;

as well as the salts thereof with inorganic or organic acids, the solvates thereof and/or the hydrates thereof.

**15.** Compound according to any one of Claims 1 to 14 of formula (I), (I'), (I") or (I"') in optically pure form.

**16.** 2-[2-(4-Cyclohexylpiperazin-1-yl)ethyl]-2-(3,4-dichlorophenyl)-4-[2-[3,5-bis(trifluoromethyl)-phenyl]acetyl]morpholine, (+) isomer, the salts thereof, the solvates thereof and/or the hydrates thereof.

**17.** 2-[2-[4-(1-Carbamoylcyclohexyl)piperidin-1-yl]ethyl]-2-(3,4-dichlorophenyl)-4-[2-[3,5-bis(trifluoromethyl)phenyl]acetyl]morpholine, (+) isomer, the salts thereof, the solvates thereof and/or the hydrates thereof.

**18.** 2-[2-[4-(1-Carbamoyl-1-methylethyl)piperazin-1-yl]ethyl]-2-(3,4-dichlorophenyl)-4-[2-[3,5-bis(trifluoromethyl)phenyl]acetyl]morpholine, (+) isomer, the salts thereof, the solvates thereof and/or the hydrates thereof.

**19.** 2-[2-[4-(1-Carbamoyl-1-methylethyl)piperidin-1-yl]ethyl]-2-(3,4-dichlorophenyl)-4-[2-[3,5-bis(trifluoromethyl)phenyl]acetyl]morpholine, (+) isomer, the salts thereof, the solvates thereof and/or the hydrates thereof.

**20.** Process for preparing the compounds of formula (I) according to Claim 1, the salts thereof, the solvates thereof and/or the hydrates thereof, **characterized in that**:

1a) a compound of formula:

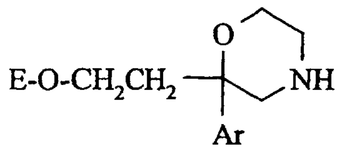

(II)

in which Ar is as defined for a compound of formula (I) in Claim 1 and E represents hydrogen or an O-protecting group, is treated with a functional derivative of an acid of formula:

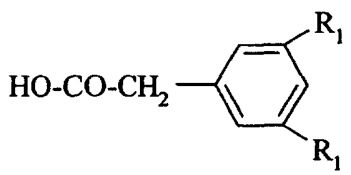

(III)

in which $R_1$ is as defined for a compound of formula (I) in Claim 1, to give a compound of formula:

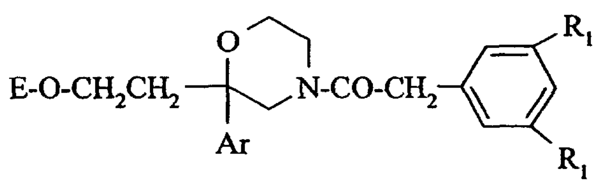

(IV) ;

2a) optionally, when E represents a protecting group, it is removed by the action of an acid or a base, to give the alcohol of formula:

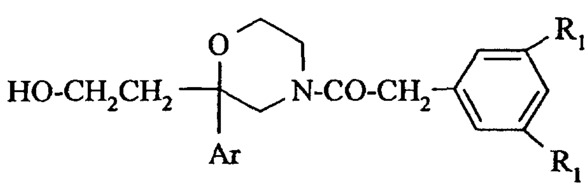

(IV, E = H) ;

3a) the alcohol obtained in step 1a) or in step 2a) of formula (IV, E = H) is treated with a compound of formula:

$$Y\text{-}SO_2\text{-}Cl \quad (V)$$

in which Y represents a methyl, phenyl, tolyl or trifluoromethyl group, to give a compound of formula:

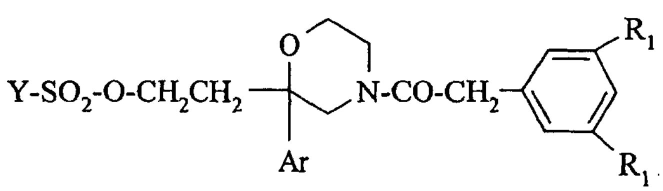

(VI)

4a) the compound of formula (VI) is reacted with a compound of formula:

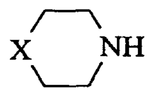 (VII)

in which X is as defined for a compound of formula (I) in Claim 1;

5a) and, optionally, the compound thus obtained is converted into one of the salts thereof with an inorganic or organic acid.

**21.** Process for preparing the compounds of formula (I) according to Claim 1, the salts thereof, the solvates thereof and/or the hydrates thereof, **characterized in that**:

1b) the compound of formula:

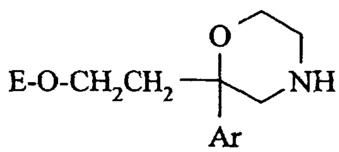 (II)

in which Ar is as defined for a compound of formula (I) in Claim 1, E represents hydrogen or an O-protecting group, is treated with a functional derivative of an acid of formula:

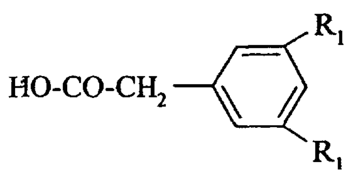 (III)

in which $R_1$ is as defined for a compound of formula (I) in Claim 1, to give a compound of formula:

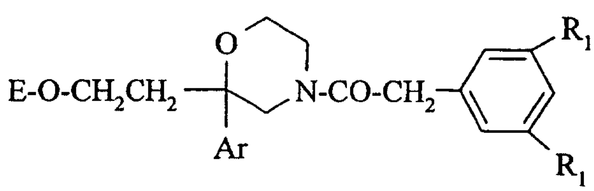 (IV) ;

optionally, when E represents a protecting group, it is removed by the action of an acid or a base, to give the alcohol of formula:

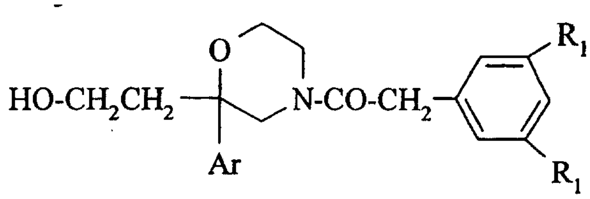 (IV, E = H) ;

2b) the compound of formula (IV, E = H) thus obtained is oxidized in order to prepare a compound of formula:

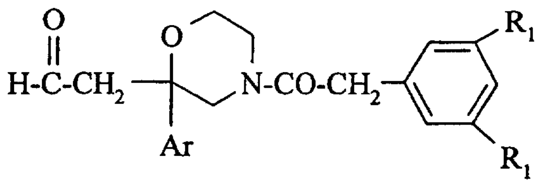

(VIII)

3b) the compound of formula (VIII) is reacted with a compound of formula:

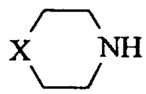

(VII)

in which X is as defined for a compound of formula (I) in Claim 1, in the presence of an acid, followed by reduction of the intermediate iminium salt formed, by means of a reducing agent;

4b) and, optionally, the compound thus obtained is converted into one of the salts thereof with an inorganic or organic acid.

**22.** Pharmaceutical composition comprising, as active principle, a compound according to any one of Claims 1 to 19 or one of the pharmaceutically acceptable salts, solvates and/or hydrates thereof.

**23.** Pharmaceutical composition according to Claim 22, containing from 0.1 to 1000 mg of active principle, in unit dosage form, in which the active principle is mixed with at least one pharmaceutical excipient.

**24.** Use of the compounds of formula (I) according to Claim 1 or one of the pharmaceutically acceptable salts, solvates and/or hydrates thereof, for the preparation of medicinal products intended for treating any pathology in which substance P and/or the human $NK_1$ receptors are involved.

**25.** Use according to Claim 24, for the preparation of medicinal products intended for treating pathologies of the respiratory, gastrointestinal, urinary, immune or cardiovascular system or of the central nervous system, as well as pain, migraine, inflammation, nausea, vomiting and skin diseases.

**26.** Use according to Claim 25, for the preparation of medicinal products intended for treating obstructive chronic bronchitis, asthma, urinary incontinence, irritable bowel syndrome, Crohn's disease, ulcerative colitis, depresssion, anxiety and epilepsy.

**27.** Medicinal products, **characterized in that** it comprises a compound according to any one of Claims 1 to 19.